# EUROPEAN PATENT SPECIFICATION

(11) **EP 1 389 637 B1**
(45) Date of publication and mention of the grant of the patent: **30.05.2012**
(21) Application number: 02017601.2
(22) Date of filing: 05.08.2002
(51) Int. Cl.: C12N 15/113, A61K 31/713, C07H 21/02, C12Q 1/68

(54) **Blunt-ended interfering RNA molecules**
Interferierende RNS Moleküle mit glatten Enden
Molécules d'ARN interférant à bords francs

(43) Date of publication of application: 18.02.2004
(73) Proprietor: Silence Therapeutics Aktiengesellschaft, 13125 Berlin (DE)
(72) Inventor: Klippel, Anke, 10779 Berlin (DE); Kaufmann, Jörg, 13437 Berlin (DE); Giese, Klaus, 10779 Berlin (DE)
(74) Representative: Bohmann, Armin K.

(56) References cited:
- WO-A-00/44895
- WO-A-01/75164
- WO-A-02/44321
- WO-A-02/055693
- PARRISH SUSAN ET AL: "Functional anatomy of a dsRNA trigger: Differential requirement for the two trigger strands in RNA interference." MOLECULAR CELL, vol. 6, no. 5, November 2000 (2000-11), pages 1077-1087, XP002226298 ISSN: 1097-2765
- ELBASHIR SAYDA M ET AL: "RNA interference is mediated by 21- and 22-nucleotide RNAs" GENES AND DEVELOPMENT, vol. 15, no. 2, 15 January 2001 (2001-01-15), pages 188-200, XP002204651 ISSN: 0890-9369
- STERNBERGER MARIA ET AL: "GeneBlocs are powerful tools to study and delineate signal transduction processes that regulate cell growth and transformation." ANTISENSE & NUCLEIC ACID DRUG DEVELOPMENT, vol. 12, no. 3, June 2002 (2002-06), pages 131-143, XP002226299 ISSN: 1087-2906
- CLEMENS JAMES C ET AL: "Use of double-stranded RNA interference in Drosophila cell lines to dissect signal transduction pathways." PROCEEDINGS OF THE NATIONAL ACADEMY OF SCIENCES OF THE UNITED STATES, vol. 97, no. 12, 6 June 2000 (2000-06-06), pages 6499-6503, XP002226300 ISSN: 0027-8424
- CZAUDERNA F ET AL: "Structural variations and stabilising modifications of synthetic siRNAs in mammalian cells", NUCLEIC ACIDS RESEARCH, vol. 31, no. 11, 1 June 2003 (2003-06-01), pages 2705-2716, XP002270732, ISSN: 0305-1048
- MA JIN-BIAO ET AL: "Structural basis for overhang-specific small interfering RNA recognition by the PAZ domain", NATURE, vol. 429, no. 6989, 20 May 2004 (2004-05-20), pages 318-322, XP002470479, ISSN: 0028-0836
- MARTINEZ J ET AL: "Single-stranded antisense siRNAs guide target RNA cleavage in RNAi", CELL, vol. 110, no. 5, 6 September 2002 (2002-09-06), pages 563-574, XP002257819, ISSN: 0092-8674

## Description

The present invention is related to a ribonucleic acid mediating RNA interference comprising a double-stranded structure whereby the double-stranded structure comprises a first strand and a second strand, whereby the first strand comprises a first stretch of contiguous nucleotides and whereby said first stretch is at least partially complementary to a target nucleic acid, and the second strand comprises a second stretch of contiguous nucleotides whereby said second stretch is at least partially identical to a target nucleic acid, the use of such ribonucleic acid, a cell and an organism, respectively, comprising such ribonucleic acid, a composition containing such ribonucleic acid and a pharmaceutical composition containing such ribonucleic acid.

RNA-mediated interference (RNAi) is a post-transcriptional gene silencing mechanism initiated by double stranded RNA (dsRNA) homologous in sequence to the silenced gene (Fire (1999), Trends Genet 15, 358-63, Tuschl, et al. (1999), Genes Dev 13, 3191-7, , Waterhouse, et al. (2001), Nature 411, 834-42, Elbashir, et al. (2001), Nature 411, 494-8, for review see Sharp (2001), Genes Dev 15, 485-90, Barstead (2001), Curr Opin Chem Biol 5, 63-6). RNAi has been used extensively to determine gene function in a number of organisms, including plants (Baulcombe (1999), Curr Opin Plant Biol 2, 109-13), nematodes (Montgomery, et al. (1998), Proc Natl Acad Sci U S A 95, 15502-7), Drosophila (Kennerdell, et al. (1998), Cell 95, 1017-26, Kennerdell, et al. (2000), Nat Biotechnol 18, 896-8). In the nematode C. elegans about one third of the genome has already been subjected to functional analysis by RNAi (Kim (2001), Curr Biol 11, R85-7, Maeda, et al. (2001), Curr Biol 11, 171-6).

Until recently RNAi in mammalian cells was not generally applicable, with the exception of early mouse development (Wianny, et al. (2000), Nat Cell Biol 2, 70-5). The discovery that transfection of duplexes of 21-nt into mammalian cells interfered with gene expression and did not induce a sequence independent interferon-driven anti-viral response usually obtained with long dsRNA led to new potential application in differentiated mammalian cells (Elbashir et al. (2001), Nature 411, 494-8). Interestingly these small interfering RNAs (siRNAs ) resemble the processing products from long dsRNAs suggesting a potential bypassing mechanism in differentiated mammalian cells. The Dicer complex, a member of the RNAse III family, necessary for the initial dsRNA processing has been identified (Bernstein, et al. (2001), Nature 409, 363-6, Billy, et al. (2001), Proc Natl Acad Sci U S A 98, 14428-33). One of the problems previously encountered when using unmodified ribooligonucleotides was the rapid degradation in cells or even in the serum-containing medium (Wickstrom (1986), J Biochem Biophys Methods 13, 97-102, Cazenave, et al. (1987), Nucleic Acids Res 15, 10507-21). It will depend on the particular gene function and assay systems used whether the respective knock down induced by transfected siRNA will be maintained long enough to achieve a phenotypic change.

International patent application WO 02/44321 discloses RNA interference mediating small RNA molecules.

Elbashir et al. (Elbashir SM et al., Genes & Development 15: 188-200, 2001) discloses that RNA interference is mediated by 21- and 22-nucleotide RNAs.

The problem underlying the present invention was to provide synthetic interfering RNA molecules which are both stable and active in a biochemical environment such as a living cell.

This problem is solved by the subject matter of the attached independent claims. Preferred embodiments may be taken from the attached dependent claims.

More specifically, in a first aspect of the present invention the problem is solved by a ribonucleic acid mediating RNA interference comprising a double stranded structure whereby the double- stranded structure comprises a first strand and a second strand, whereby the first strand comprises a first stretch of contiguous nucleotides and whereby said first stretch is at least partially complementary to a target nucleic acid, and the second strand comprises a second stretch of contiguous nucleotides whereby said second stretch is at least partially identical to the target nucleic acid, whereby the double stranded structure is blunt ended on both sides of the double strand and the length of said first strand and said first stretch and the length of said second strand and said second stretch is 18 or 19 bases, for use in a method for the treatment of a disease.

In an embodiment of the ribonucleic acid according to the present invention at least one nucleotide of the ribonucleic acid has a modification at the 2'-position and the modification is preferably selected from the group comprising amino, fluoro, methoxy and alkyl.

In an embodiment of the ribonucleic acid according to the present invention the complementarity between said first strand and the target nucleic acid is perfect.

In an embodiment of the ribonucleic acid according to the present invention the duplex formed between the first strand and the target nucleic acid comprises at least 15 nucleotides wherein there is one mismatch or two mismatches between said first strand and the target nucleic acid forming said double-stranded structure.

In a preferred embodiment of the ribonucleic acid according to the present invention the target gene is selected from the group comprising structural genes, housekeeping genes, transcription factors, motility factors, cell cycle factors, cell cycle inhibitors, enzymes, growth factors, cytokines and tumor suppressors.

In an embodiment the disease is selected from the group comprising glioblastoma, prostate cancer, breast cancer, lung cancer, liver cancer, colon cancer, pancreatic cancer and leukaemia, diabetes, obesity, cardiovascular diseases, and metabolic diseases.

In a second aspect the problem underlying the present invention is solved by the use of a ribonucleic acid according to any of the aspects of the present invention, for the manufacture of a medicament.

In an embodiment of the use according to the second aspect of the present invention the medicament is for the treatment of a disease or of a condition which is selected from the group comprising glioblastoma, prostate cancer, breast cancer, lung cancer, liver cancer, colon cancer, pancreatic cancer and leukaemia, diabetes, obesity, cardiovascular diseases, and metabolic diseases.

In a third aspect the problem underlying the present invention is solved by a cell, preferably a knockdown cell, containing a ribonucleic acid according to the first aspect of the present invention whereby if the cell is a human cell, the human cell is an isolated human cell.

In a fourth aspect the problem underlying the present invention is solved by an organism, preferably a knockdown organism, containing a ribonucleic acid according to the first aspect of the present invention wherein the organism is different from a human being.

In a fifth aspect the problem underlying the present invention is solved by a composition containing a ribonucleic acid according to the first aspect of the present invention.

In a sixth aspect the problem underlying the present invention is solved by a pharmaceutical composition containing a ribonucleic acid according to the first aspect of the present invention, and a pharmaceutically acceptable carrier.

Also disclosed is a method for inhibiting the expression of a target gene in a cell or derivative thereof comprising introduction of a ribonucleic acid according to any of the aspects of the present invention into the cell in an amount sufficient to inhibit expression of the target gene, wherein the target gene is the target gene of the a ribonucleic acid according to any of the aspects of the present invention.

The present invention is based on the surprising finding that small interfering RNAs can be designed such as to be both highly specific and active as well as stable under the reaction conditions typically encountered in biological systems such as biochemical assays or cellular environments. The various interfering RNAs described in the prior art such as by Tuschl et al. (international patent application WO 01/75164) provide for a length of 21 to 23 nucleotides and a modification at the 3' end of the double-stranded RNA. It has been surprisingly found by the present inventors that the problem of stability of interfering RNA, including small interfering RNA (siRNA) which is generally referred to herein in the following as RNAi, actually resides in the attack of endonucleases rather than exonucleases as thought earlier. Based on this finding several strategies have been perceived by the present inventors which are subject to the present application.

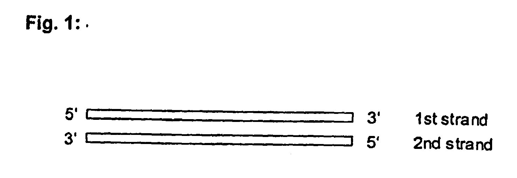

The present invention is thus related to new forms of interfering RNA. RNAi consists of a ribonucleic acid comprising a double-stranded structure. Said double-stranded structure is formed by a first strand and a second strand. Said first strand comprises a stretch of contiguous nucleotides, also referred to as first stretch of contiguous nucleotides herein, and this first stretch is at least partially complementary to a target nucleic acid. Said second strand comprises also a stretch of contiguous nucleotides whereby said second stretch is at least partially identical to a target nucleic acid. The very basic structure of this ribonucleic acid is schematically shown in Fig. 1. Said first strand and said second strand are preferably hybridised to each other and form the double-stranded structure. The hybridisation typically occurs by Watson Crick base pairing. Depending on the particular sequence of the first stretch and the second stretch, the hybridisation or base pairing is not necessarily complete or perfect, which means that the first and the second stretch_are not 100 % base paired due to mismatches. There might also be one or more mismatches within the duplex. Said mismatches have no effect on the RNAi activity if placed outside a stretch of 17 matching nucleotides. If mismatches are placed to yield only 15 or less contiguous matching nucleotides, the RNAi molecule typically shows a reduced activity in down regulating mRNA for a given target compared to a 17 matching nucleotide duplex.

The first stretch of contiguous nucleotides is essentially complementary to a target nucleic acid, more preferably to a part of the target nucleic acid. Complementary as used herein preferably means that the nucleotide sequence of the first strand is hybridising to a nucleic acid sequence or a part thereof of a target nucleic acid sequence. Typically, the target nucleic acid sequence is, in accordance with the mode of action of interfering ribonucleic acids, a single stranded RNA, more preferably an mRNA. Such hybridisation occurs most likely through Watson Crick base pairing, however, is not necessarily limited thereto. The extent to which said first strand and more particularly the first stretch of contiguous nucleotides of said first strand is complementary to a target nucleic acid sequence can be as high as 100% and be as little as 80%, preferably 80-100%, more preferably 85-100%, most preferably 90-100%. Optimum complementarity seems to be 95-100%. Complementarity in this sense means that the aforementioned range of nucleotides, such as, e. g., 80%-100%, depending on the particular range, of the nucleotides are perfect by Watson Crick base pairing. It is shown in one aspect of the present invention that the complementarity between said first stretch of nucleotides and the target RNA has to be 18-19 nucleotides, stretches of as little as 17 nucleotides even with two sequence specific overhangs are not functional in mediating RNAi. Accordingly, given a duplex having a length of 19 nucleotides or base pairs a minimum complementarity of 17 nucleotides or nucleotide base pairs would be acceptable allowing for a mismatch of two nucleotides. It is disclosed that in case of a duplex consisting of 20 nucleotides or base pairs a complementarity of 17 nucleotides or nucleotide base pairs would be allowable and functionally active. The same applies to a duplex of 21 nucleotides or base pairs with a total of 17 complementary nucleotides or base pairs. Basically, the extent of complementarity required for a length of a duplex, i. e. of a double stranded structure, can also be based on the melting temperature of the complex formed by either the double stranded structure as described herein or by the complex of the first stretch of the first strand and the target nucleic acid.

It is to be understood that all of the ribonucleic acid of the present invention are suitable to cause or being involved in RNA mediated interference such as, for example, described in international patent applications WO 99/32619, WO 00/44895 and WO 01/75164.

Also disclosed is a strategy according to which an interfering ribonucleic acid molecule may be designed according to the present invention is to have an optimum length of 18 or 19 nucleotides of the stretch which is complementary to the target nucleic acid. It is also within the present invention that said optimum length of 18 or 19 nucleotides is the length of the double stranded structure in the RNAi used. This length requirement is clearly different from the technical teaching of the prior art such as, for example, the international patent application WO 01/75164. It is within the present invention that any further design, both according to the present invention and as described in the prior art, can be realised in connection with an interfering ribonucleic acid having said length characteristics, i.e. a length of 18 or 19 nucleotides.

It is within the present invention the interfering ribonucleic acid molecule may be designed is to have a free 5' hydroxyl group, also referred to herein as free 5' OH-group at the first strand. A free 5' OH-group means that the most terminal nucleotide forming the first strand is present and is thus not modified, particularly not by an end modification. Typically, the terminal 5'-hydroxy group of the second strand, respectively, is also present in an unmodified manner. In a more preferred embodiment, also the 3'-end of the first strand and first stretch, respectively, is unmodified such as to present a free OH-group which is also referred to herein as free 3'OH-group. Preferably such free OH-group is also present at the 3'-end of the second strand and second stretch, respectively. In other embodiments of the ribonucleic acid molecules according to the present invention the 3'-end of the first strand and first stretch, respectively, and/or the 3'-end of the second strand and second stretch, respectively, may have an end modification at the 3' end.

As used herein the terms free 5'OH-group and 3'OH-group also indicate that the respective most terminal nucleotide at the 5' end and the 3' end of the polynucleotide, respectively, presents a OH-group. Such OH-group may stem from either the sugar moiety of the nucleotide, more preferably from the 5'position in case of the 5'OH-group and from the 3'position in case of the 3'OH-group, or from a phosphate group attached to the sugar moiety of the respective terminal nucleotide. The phosphate group may in principle be attached to any OH-group of the sugar moiety of the nucleotide. Preferably, the phosphate group is attached to the 5'OH-group of the sugar moiety in case of the free 5'OH-group and/or to the 3'OH-group of the sugar moiety in case of the free 3'OH-group.

As used herein with any strategy for the design of RNAi or any embodiment of RNAi disclosed herein, the term end modification means a chemical entity added to the most 5' or 3' nucleotide of the first and/or second strand. Examples for such end modifications include, but are not limited to, inverted (deoxy) abasics, amino, fluoro, chloro, bromo, CN, CF, methoxy, imidazole, caboxylate, thioate, C₁ to C₁₀ lower alkyl, substituted lower alkyl, alkaryl or aralkyl, OCF₃, OCN, O-, S-, or N-alkyl; O-, S-, or N-alkenyl; SOCH₃; SO₂CH₃; ONO₂; NO₂, N₃; heterozycloalkyl; heterozycloalkaryl; aminoalkylamino; polyalkylamino or substituted silyl, as, among others, described in European patents EP 0 586 520 B1 or EP 0 618 925 B1.

A further end modification is a biotin group. Such biotin group may preferably be attached to either the most 5' or the most 3' nucleotide of the first and/or second strand or to both ends. In a more preferred embodiment the biotin group is coupled to a polypeptide or a protein. It is also within the scope of the present invention that the polypeptide or protein is attached through any of the other aforementioned end modifications. The polypeptide or protein may confer further characteristics to the inventive nucleic acid molecules. Among others the polypeptide or protein may act as a ligand to another molecule. If said other molecule is a receptor the receptor's function and activity may be activated by the binding ligand. The receptor may show an internalization activity which allows an effective transfection of the ligand bound inventive nucleic acid molecules. An example for the ligand to be coupled to the inventive nucleic acid molecule is VEGF and the corresponding receptor is the VEGF receptor.

Various possible embodiments of the RNAi of the present invention having different kinds of end modification(s) are presented in the following table 1.

**Table 1: Various embodiments of the interfering ribonucleic acid according to the present invention**

| | | **1^{st} strand/1^{st} stretch** | **2^{nd} strand/ 2nd stretch** |
|---|---|---|---|
| **1.)** | **5'-end** | free OH | free OH |
| | **3'-end** | free OH | free OH |
| | | | |
| **2.)** | **5'-end** | free OH | free OH |
| | **3'-end** | end modification | end modification |
| | | | |
| **3.)** | **5'-end** | free OH | free OH |
| | **3'-end** | free OH | end modification |
| | | | |
| **4.)** | **5'-end** | free OH | free OH |
| | **3'-end** | end modification | free OH |
| | | | |
| **5.)** | **5'-end** | free OH | end modification |
| | **3'-end** | free OH | free OH |
| | | | |
| **6.)** | **5'-end** | free OH | end modification |
| | **3'-end** | end modification | free OH |
| | | | |
| **7.)** | **5'-end** | free OH | end modification |
| | **3'-end** | free OH | end modification |
| | | | |
| **8.)** | **5'-end** | free OH | end modification |
| | **3'-end** | end modification | end modification |

The various end modifications as disclosed herein are preferably located at the ribose moiety of a nucleotide of the ribonucleic acid. More particularly, the end modification may be attached to or replace any of the OH-groups of the ribose moiety, including but not limited to the 2'OH, 3'OH and 5'OH position, provided that the nucleotide thus modified is a terminal nucleotide. Inverted abasics are nucleotides, either desoxyribonucleotides or ribonucleotides which do not have a nucleobase moiety. This kind of compound is, among others, described in Sternberger et al.(2002), Antisense. Nucl. Ac. Drug Dev. in press.

Any of the aforementioned end modifications may be used in connection with the various embodiments of RNAi depicted in table 1. In connection therewith it is to be noted that any of the RNAi forms or embodiments disclosed herein with the sense strand being inactivated, preferably by having an end modification more preferably at the 5' end, are particularly advantageous. This arises from the inactivation of the sense strand which corresponds to the second strand of the ribonucleic acids described herein, which might otherwise interfere with an unrelated single-stranded RNA in the cell. Thus the expression and more particularly the translation pattern of the transcriptome of a cell is more specifically influenced. This effect is also referred to as off-target effect. Referring to table 1 those embodiments depicted as embodiments 7 and 8 are particularly advantageous in the above sense as the modification results in an inactivation of the ― target unspecific ― part of the RNAi (which is the second strand) thus reducing any unspecific interaction of the second strand with single-stranded RNA in a cellular or similar system where the RNAi according to the present invention is going to be used to knock down specific ribonucleic acids and proteins, respectively.

In accordance with the present invention the ribonucleic acid comprises a double-stranded structure whereby the double-stranded structure comprises a first strand and a second strand, whereby the first strand comprises a first stretch of contiguous nucleotides and whereby said first stretch is at least partially complementary to a target nucleic acid, and the second strand comprises a second stretch of contiguous nucleotides whereby said second stretch is at least partially identical to a target nucleic acid whereby the double-stranded structure is blunt-ended. As used in connection with the present description the term double-stranded structure is also referred to as duplex. This design of RNAi is thus clearly different from, e. g., the one of Tuschl et al. as specified in international patent application WO 01/75164 which discloses a 3'-overhang. As used herein overhang refers to a double-stranded structure whereby at least one end of one strand is longer than the corresponding end of the other strand forming the double-stranded structure, which is also referred to herein as the counter strand. Preferably, the first stretch is identical to the first strand and the second stretch is identical to the second strand.

Taken the effectiveness of blunt-ended RNAi and the advantages of an end modification of either the first or the second strand or both, respectively, of the respective ribonucleic acid it is preferred to have a combination of both design principles. In other words, it is within the present invention to have blunt-ended RNAi carrying any end modification scheme as depicted in table 1.

Also disclosed is a strategy according to which the ribonucleic acid has an overhang at the 5'-end of the ribonucleic acid. More particularly, such overhang may in principle be present at either or both the first strand and second strand of the ribonucleic acid. The length of said overhang may be as little as one nucleotide and as long as 2 to 8 nucleotides, preferably 2, 4, 6 or 8 nucleotides. The 5' overhang may be located on the first strand and/or the second strand of the ribonucleic. acid The nucleotide(s) forming the overhang may be (a) desoxyribonucleotide(s), (a) ribonucleotide(s) or a continuation thereof.

The overhang preferably comprises at least one desoxyribonucleotide, whereby said one desoxyribonucleotide is preferably the most 5'-terminal one. The 3'-end of the respective counter-strand of the ribonucleic acid does not have an overhang, more preferably not a desoxyribonucleotide overhang. Here again, any of the ribonucleic acids may comprise an end modification scheme as outlined in connection with table 1 and/or and end modification as outlined herein.

In accordance with the present invention the ribonucleic acids of the present invention form a certain pattern of modified nucleotides on at least one of the strands and more particularly on one of the stretches of contiguous nucleotides of the ribonucleic acid(s) according to the present invention. The kind of modification of said nucleotides may be the same as discussed in connection with the other strategies for designing interfering RNA as disclosed herein and more particularly the kind of modification described herein for or as an end modification, such as, e.g., inverted abasics, methoxy, or amino and the like at the ribose moiety of at least one nucleotide forming the ribonucleotide acids according to the present application. It is to be noted that the modification of said nucleotides may be any form of modification described herein, more particularly the kind of modification as described herein as end modification with the particularity that the so-called end modification is not necessarily located at terminal nucleotides. Rather the modification is occurring at a non-terminal nucleotide. Under such conditions the modification is preferably attached to the ribose moiety of the - to be - modified nucleotide and even more preferably to the 2'position of the ribose moiety.

It is also within the present invention that any ribonucleic acid designed according to this strategy may also have the features conferred to a ribonucleic acid according to the present invention by any of the other design strategies disclosed herein. Accordingly, the interfering ribonucleic acid having a pattern of modified nucleotides may have an end modification, an end modification scheme, or any combination of these elements or characteristics.

Apart from the aforementioned modifications which may be presented either as end modifications or as modification pattern, the ribonucleic acid backbone as such may be further modified by forming different links between the nucleotides. Such different links are, among others, described in European patent EP 0 586 520 B1 and European patent EP 0 618 925 B1. Of particular interest here are internal modification(s) of the ribonucleic acid backbone which have been shown to confer higher nuclease resistance of ribooligonucleotides. In a preferred embodiment the modification of the modified nucleotide is a methoxylation of the 2'-OH-group of the ribose moiety of the nucleotide.

In a preferred embodiment, both strands, and more particularly both the first stretch and the second stretch show this kind of modification of the nucleotides forming said strands and stretches, respectively. However, it is also within the present invention that either the first strand and first stretch, respectively, or the second strand and second stretch, respectively, show this particular pattern of modification of the nucleotides. As used herein, the term group of modified nucleotide or flanking group of nucleotide may comprise or represent as little nucleotides as one nucleotide.

Taken the stretch of contiguous nucleotides there is a pattern of modification of the nucleotides forming the stretch may be realised such that a single nucleotide or group of nucleotides which are covalently linked to each other via standard phosphorodiester bonds or, at least partially, through phosphorothioate bonds, show such kind of modification. In case such nucleotide or group of nucleotides is not forming the 5'-end or 3'-end of said stretch a nucleotide or group of nucleotides follows on both sides of the nucleotide which does not have the modification of the preceding nucleotide or group of nucleotides. It is to be noted that this kind of nucleotide or group of nucleotides, however, may have a different modification. This sequence of modified nucleotide and group of nucleotides, respectively, and unmodified or differently modified nucleotide or group of nucleotides may be repeated one or several times. Preferably, the sequence is repeated more than one time. For reason of clarity the pattern is discussed in more detail in the following, generally referring to a group of modified nucleotides or a group of unmodified nucleotides whereby each of said group may actually comprise as little as a single nucleotide. Unmodified nucleotide as used herein means either not having any of the afore-mentioned modifications at the nucleotide forming the respective nucleotide or group of nucleotides, or having a modification which is different from the one of the modified nucleotide and group of nucleotides, respectively.

It is also within the present invention that the modification of the unmodified nucleotide(s) wherein such unmodified nucleotide(s) is/are actually modified in a way different from the modification of the modified nucleotide(s), can be the same or even different for the various nucleotides forming said unmodified nucleotides.

The pattern of modified and unmodified nucleotides may be such that the 5'-terminal nucleotide of the strand or of the stretch starts with a modified group of nucleotides or starts with an unmodified group of nucleotides. However, in an alternative embodiment it is also possible that the 5'-terminal nuleotide is formed by an unmodified group of nucleotides.

This kind of pattern may be realised either on the first stretch or the second stretch of the interfering RNA or on both. It has to be noted that a 5' phosphate on the target-complementary strand of the siRNA duplex is required for siRNA function, suggesting that cells check the authenticity of siRNAs through a free 5' OH (which can be phosphorylated) and allow only such bona fide siRNAs to direct target RNA destruction (Nykanen, et al. (2001), Cell 107, 309-21).

Preferably, the first stretch shows a kind of pattern of modified and unmodified groups of nucleotides, whereas the second stretch does not show this kind of pattern. This may be useful insofar as the first stretch is actually the more important one for the target-specific degradation process underlying the interference phenomenon of RNA so that for specificity reasons the second stretch can be chemically modified so it is not functional in mediating RNA interference.

However, it is also within the present invention that both the first stretch and the second stretch have this kind of pattern. Preferably, the pattern of modification and non-modification is the same for both the first stretch and the second stretch.

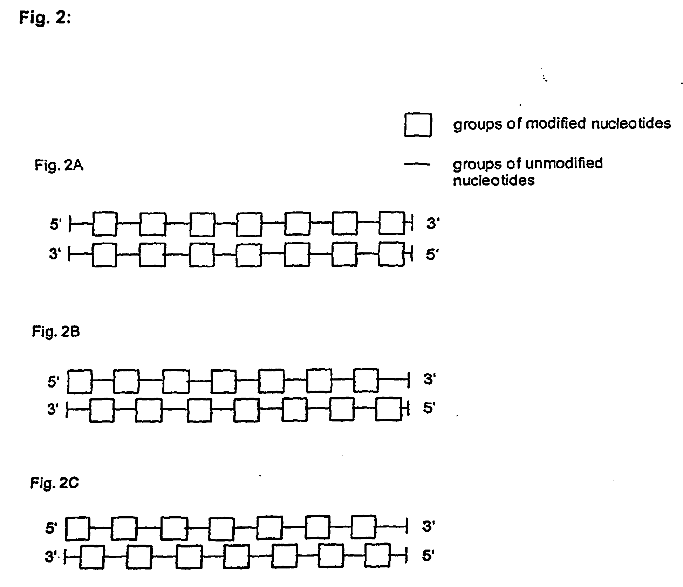

In a preferred embodiment the group of nucleotides forming the second stretch and corresponding to the modified group of nucleotides of the first stretch are also modified whereas the unmodified group of nucleotides of or forming the second stretch correspond to the unmodified group of nucleotides of or forming the first stretch. This possibility is schematically depicted in Fig. 2A. Another alternative is that there is a phase shift of the pattern of modification of the first stretch and first strand, respectively, relative to the pattern of modification of the second stretch and second strand, respectively. Preferably, the shift is such that the modified group of nucleotides of the first strand corresponds to the unmodified group of nucleotides of the second strand and vice versa. This possibility is shown in Fig. 2B. It is also within the present invention that the phase shift of the pattern of modification is not complete but overlapping as illustrated in Fig. 2C.

In a preferred embodiment the second nucleotide at the terminus of the strand and stretch, respectively, is an unmodified nucleotide or the beginning of group of unmodified nucleotides. Preferably, this unmodified nucleotide or unmodified group of nucleotides is located at the 5'-end of the first and second strand, respectively, and even more preferably of the first strand. In an even more preferred embodiment the unmodified nucleotide or unmodified group of nucleotide is located at the 5'-end of the first strand and first stretch, respectively. In a preferred embodiment the pattern consists of alternating single modified and unmodified nucleotides.

Although not limited thereto, the double-stranded structure of the inventive ribonucleic acid, which is also referred to as duplex, is formed by the first strand and second strand, respectively, or the first and second stretch of contiguous nucleotides. The length of the first stretch and second stretch, respectively, is 18 or 19 bases. In connection with this it is to be noted that a length of less than 30 nucleotides, preferably less than 21 nucleotides does not cause any biological system which is basically capable of showing RNA interference and also interferon response, to develop an interferon response. The reason for this resides in the observation that a given cell is experiencing profound physiological changes when double-stranded DNA longer than 30 base pairs binds and activates the protein kinase PKR and 2',5'-oligoadenylate synthetase. Activated PKR stalls translation via phosphorylation of eIF2a, activated 2',5'-AS causes mRNA degradation. These effects are not desired in target validation and animal models because they overrule the effect of the target specific knockdown on the phenotype.

Also disclosed is a strategy for the design of interfering ribonucleic acids according to which the ribonucleic acid comprises a double-stranded structure whereby the double-stranded structure comprises a first strand and a second strand, whereby the first strand comprises a first stretch of contiguous nucleotides and whereby said first stretch is at least partially complementary to a target nucleic acid, and the second strand comprises a second stretch of contiguous nucleotides whereby said second stretch is at least partially identical to a target nucleic acid whereby one terminus of the first strand and one terminus of the second strand are linked by a loop structure.

In an embodiment the loop structure is comprised of a non-nucleic acid polymer. Such non-nucleic acid polymer may be polyethylene glycol or similar polymers The non-nucleic acid polymers may in principle be chosen from the group comprising polymers which do not comprise a polynucleotide and allow that the two strands to be linked may actually hybridize to each other. To allow for such hybridization the molecule or moiety of the molecule linking the two stretches hybridizing with each other, has to have a certain molecular structure or molecular flexibility to allow the bending of the molecule so as to allow that both stretches get in close contact and in a three-dimensional orientation which permits hybridization. Such molecule or moiety factually acts as a hinge. Besides polyethylene glycol amino acid based molecules may be used. Such amino acid based molecules may be either homopolymers or hetereopolymers. A useful example is a homopolymer consisting of seven glycine residues which allows the generation of a hinge as required to bring the two stretches to hybridize in the close proximity as needed. This glycine based hinge is described, e. g., in Guan K. L. and Dixon J. E. (1991), Anal Biochem. 192, 262. In another embodiment the hinge may be formed by crown ethers known in the art.

In an alternative the loop is comprised of a nucleic acid. As used herein, LNA as described in Elayadi and Corey (2001)Curr Opin Investig Drugs. 2(4):558-61. Review; Orum and Wengel (2001) Curr Opin Mol Ther. 3(3):239-43; and PNA are regarded as nucleic acids and may also be used as loop forming polymers. Basically, the 5'-terminus of the first strand may be linked to the 3'-terminus of the second strand. As an alternative, the 3'-end of the first strand may be linked to the 5'-terminus of the second strand. The nucleotide sequence forming said loop structure is regarded as not being critical. However, the length of the nucleotide sequence forming such loop seems to be critical for sterical reasons. Accordingly, a minimum length of four nucleotides seems to be appropriate to form the required loop structure. In principle, the maximum number of nucleotides forming the hinge or the link between both stretches to be hybridized is not limited. However, the longer a polynucleotide is, the more likely secondary and tertiary structures are formed and thus the required orientation of the stretches affected. Preferably, a maximum number of nucleotides forming the hinge is about 12 nucleotides. It is within the disclosure of this application that any of the designs described above may be combined with the present sixth strategy, i. e. by linking the two strands covalently in a manner that back folding (loop) can occur through a loop structure.

The present inventors have surprisingly found that if the loop is placed 3' of the antisense strand, i. e. the first strand of the ribonucleic acid(s) according to the present invention, the activities of this kind of RNAi are higher compared to the placement of the loop 5' of the antisense strand. Accordingly, the particular arrangement of the loop relative to the antisense strand and sense strand, i. e. the first strand and the second strand, respectively, is crucial and is thus in contrast to the understanding as expressed in the prior art where the orientation is said to be of no relevance. However, this seems not true given the experimental results presented herein. The understanding as expressed in the prior art is based on the assumption that any RNAi, is subject to a processing during which non-loop linked RNAi is generated. However, if this was the case, the clearly observed increased activity of those structures having the loop placed 3' of the antisense could not be explained. Insofar a preferred arrangement in 5' -> 3' direction of this kind of small interfering RNAi is second strand - loop - first strand. The respective constructs may be incorporated into suitable vector systems. Preferably the vector comprises a promoter for the expression of RNAi. Preferably the respective promoter is pol III and more preferably the promoters are the U6, H1, 7SK promoter as described in Good et al. (1997) Gene Ther, 4, 45-54.

Because of the general applicability of the concept of interfering RNA and thus the knockdown or knockout for a coding nucleotide such as an mRNA any gene producing such RNA may be modified in its expression by using the ribonucleic acid molecule according to the present invention. Because of this basic and generally applicable mechanism any application based thereon can be realised which imply the knockdown or knockout of a gene. A preferred application is the use of the inventive ribonucleic acid for target validation. As used herein, target validation shall mean a process that involves taking steps to prove that a DNA, RNA, or protein molecule is directly involved in a biological process, preferably in a process ―causally ― involved in a disease or non-standard condition and is therefore a suitable target for development of a new therapeutic compound. Sequence homology studies have successfully classified genes into target families. The enormous task of deciphering which of these targets are key players in diseases and which should be used for subsequent drug development needs to be addressed in an efficient manner. Therefore, the knockdown of gene expression should be reduced by 50-100%, preferably by 90% to see significant effects on the phenotype. In other cases depending on the gene, a knockdown of as little as 20% might be sufficient to yield a phenotype. A phenotype will be defined by comparison of cells containing functional RNAi molecules with cells containing non functional RNAi molecules. This will ensure a significant readout even under conditions where the protein function is inhibited only partially. Generally there is no linear correlation between degree of mRNA reduction and the extent of the phenotype. It has to be acknowledged that for some proteins a reduction of about 20 % of the protein is sufficient to create a change in the phenotype whereas in case of other genes and mRNA, respectively, as little as 5 to 10 % remaining protein is sufficient to maintain the observed phenotype.

A further use of the ribonucleic acid molecules according to the present invention is its use for the manufacture of a medicament or its use as a medicament. Such medicament could either be used for diseases or conditions such as any type of cancer where a gene and its product have been linked to the onset, cause or progression of this disease. Additionally, such medicament could be used to treat diseases where the presence or overexpression of a gene product is causing a pathological phenotype. In a preferred embodiment the disease is characterised by a gain of function and may be remedied through application or administration of the corresponding, biologically active RNAi. Diseases or conditions which may be treated by the medicament comprising a ribonucleic acid as disclosed herein, may be selected from the group comprising cancer, heart diseases, metabolic diseases, dermatological diseases, inflammatory diseases, immune system disorders and autoimmune disorders. The various forms of cancer include, but are not limited to, solid tumors and tumors of the hematopoietic system, such as glioblastoma, prostate cancer, breast cancer, lung cancer, liver cancer, pancreatic cancer and leukaemia. Metabolic diseases include, but are not limited to, obesity and diabetes. Dermatological diseases include, but are not limited to, psoriasis. In another aspect the ribonucleic acid molecules according to the present invention may be used as diagnostics, preferably for those diseases as specified in connection with the above-mentioned diseases and conditions. Such diagnosis could be based on the observation that upon applying the ribonucleic acid molecules according to the present invention to a sample a change in the expression pattern of the sample occurs. Preferably such sample comprises cells from a subject from whom it is assumed that it may exhibit said disease or condition to be treated.

Also disclosed is a further application of the nucleic acids according to the present invention which is their use in the screening of pharmaceutically active compounds and lead optimization. The latter is done such as to compare the effect of candidate drugs such as small molecules and compare the effect created by said candidate drugs with the effect observed upon administering specific RNAi designed on the basis of the principles disclosed herein. In doing so candidate drugs having off-target effects may be eliminated from the screening process whereas those candidate drugs which create a similar or identical phenotype are deemed as highly relevant lead compound or may even be a pharmaceutically active compound themselves. In this approach, the highly specific RNAi molecules act as gold standard against which candidate drugs are measured.

In a further aspect the invention is related to a cell, preferably a knockdown cell, which contains a ribonucleic acid as disclosed herein. Such cell is preferably a cell which is either isolated or contained in a tissue or even organ which again preferably is not contained in an organism. However, the cell may also be contained in an organism. The cell is preferably a cell which is involved in the disease or condition which is to be treated by the inventive ribonucleic acids. This kind of knock-down cells may be used to generate an expression profile based on, e. g., mRNA or protein, in order to elucidate functional relationship and to determine downstream targets.

In a further aspect the invention is related to an organism containing a ribonucleic acid as disclosed herein. Preferably such organism is a vertebrate organism and more preferably the vertebrate organism is a mammal. A mammal as used herein is, among others and not limited thereto, an ape, a dog, a cat, a goat, a sheep, a pig, a guinea pig, a rabbit, a mouse, and a rat. In a further aspect the present invention is related to a composition containing a ribonucleic acid according to the present invention. Preferably such composition comprises negative and positive controls either in combination with the effective ribonucleic acid or separated therefrom. Such composition may further comprise a solvent, preferably a buffer.

In a further aspect the present invention is related to a pharmaceutical composition containing a ribonucleic acid according to the present invention and a pharmaceutically acceptable carrier. Pharmaceutically acceptable carriers are known to the one skilled in the art and comprise, among others, diluent, buffers and the like. The pharmaceutical composition may comprise further pharmaceutically active compounds. In case the disease or condition to be treated using the ribonucleic acid molecules according to the present invention are preferably those which are already now used in connection with the treatment of said diseases or conditions. Due to the different mode of action of the ribonucleic acid molecules according to the present invention and the medicaments used for the treatment of said diseases and conditions according to the prior art, synergistic effects will happen.

The invention as defined by the claims is now further illustrated by reference to the figures and examples from which further features, embodiments and advantages of the present invention may be taken.

Fig. 1 shows a schematic illustration defining the terminology as used herein. The upper of the two strand is the first strand and the antisense strand of the targeted nucleic acid such as mRNA. The second strand is the one which essentially corresponds in its sequence to the targeted nucleic acid and thus forms the sense strand. Both, the first strand and second strand form a double-stranded structure, typically through Watson Crick base pairing.

Fig. 2 illustrates some embodiments of the ribonucleic acid molecules of the present invention with patterns of modified and unmodified groups of nucleotides which is also referred to herein as pattern of modification. In Fig. 2A the modified and unmodified groups of nucleotides on both the first stretch and the second stretch are located on corresponding parts of the stretches whereas in Fig. 2B the pattern realised on the first strand is also realised on the second strand, however, with a phase shift such that the modified group of nucleotides of the first stretch is base pairing with an unmodified group of nucleotides of the second stretch and vice versa. In Fig. 2C a further possibility of arranging the modified and unmodified groups of nucleotides is realised. It is also within the present invention that the pattern of the first stretch is independent from the pattern of the second stretch and that both patterns partially overlap in terms of relative position to each other in the double-stranded structure defined by base pairing.

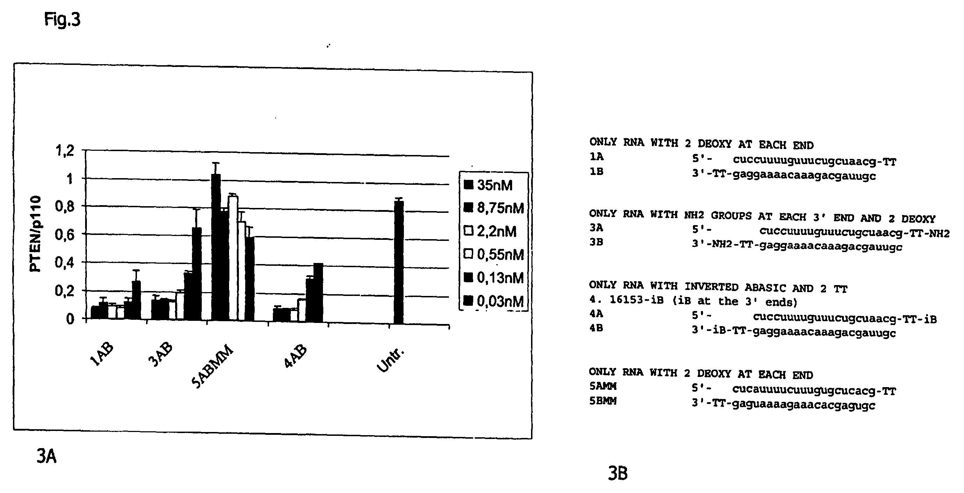

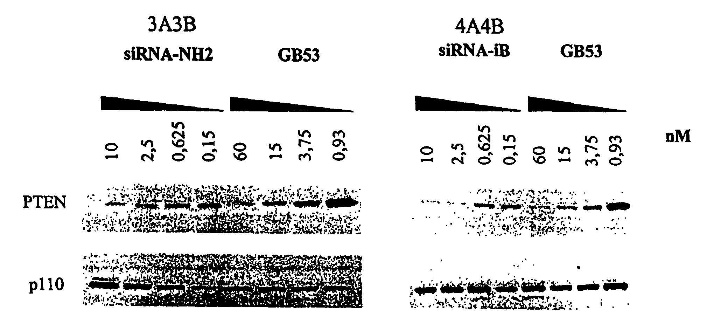

Fig. 3 shows the result of a knockdown experiment using RNAi molecules with different end protection groups. More particularly Fig. 3A shows that the various forms of end protected RNAi molecules are functional on the knockdown of PTEN mRNA.

Fig. 3B shows the sequence of the different RNAi molecules used in the experiment the result of which is depicted in Fig. 3A. Fig. 3C shows the result of an immunoblot analysis of PTEN protein after treatment with modified RNAi molecules in comparison to PTEN specific antisense constructs.

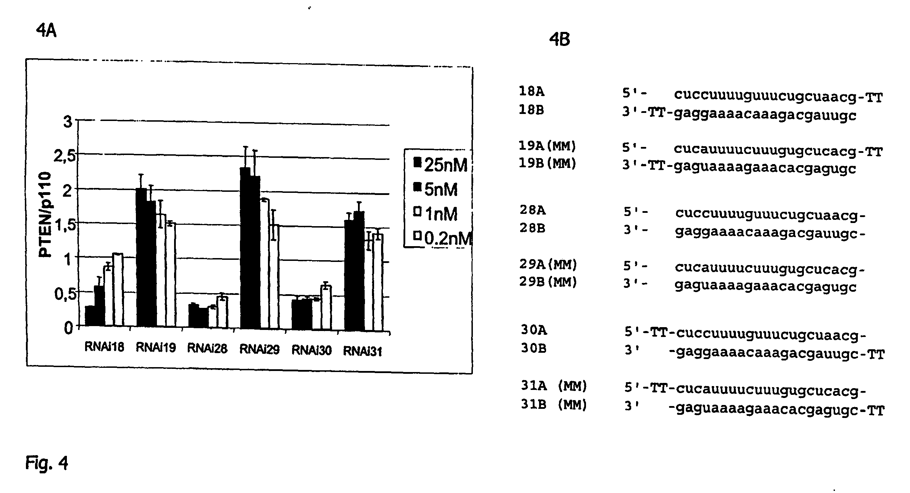

Fig. 4 shows that the 3' overhang of RNAi molecules is not important for RNA interference. More particularly, Fig. 4A shows a dose response curve of different RNAi molecules and Fig. 4B shows the sequence of the RNAi molecules used in the experiment the result of which is shown in Fig. 4A.

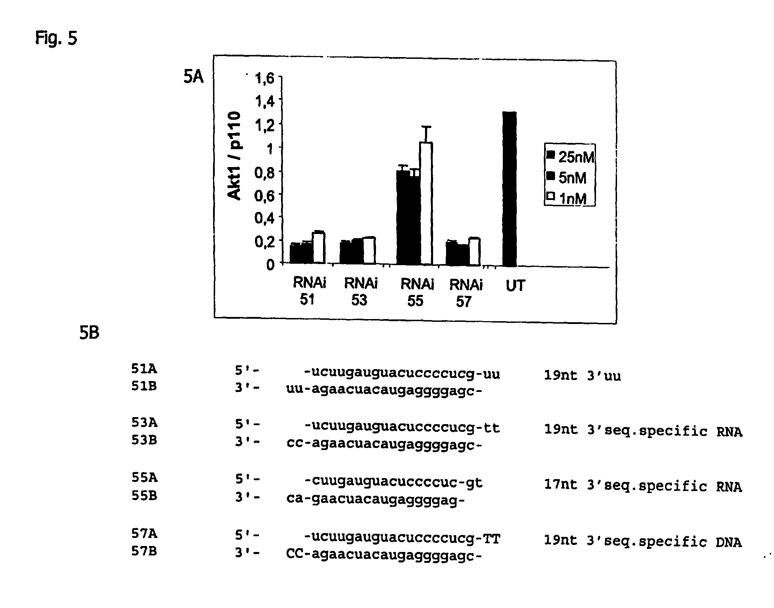

Fig. 5 shows that duplex length of the RNAi molecules has to be at least 18-19 nucleotide. More particularly, Fig. 5B shows the sequence of the PTEN specific RNAi molecules used in the experiment the result of which is depicted in Fig. 5A as dose response curve.

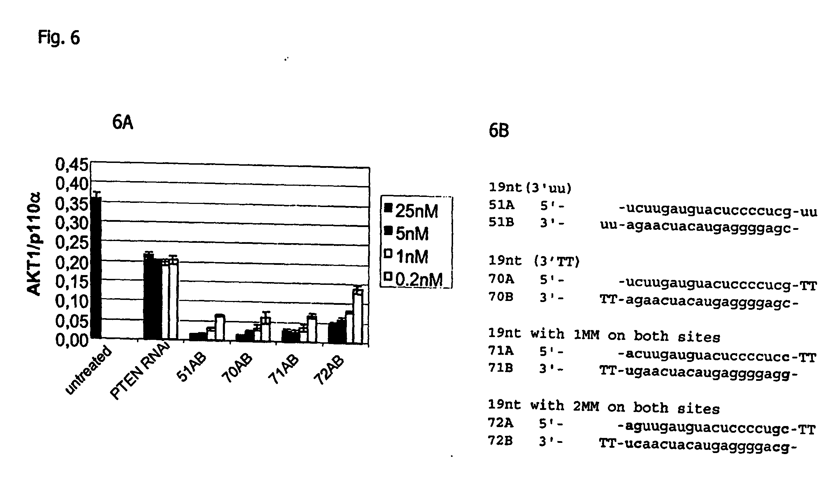

Fig. 6 shows that four terminal mismatched nucleotides in RNAi molecules with a length of 19 nucleotides are still functional in mediating Akt1 knockdown. More particularly, Fig. 6B shows the sequence of the RNAi molecules used in the experiment the result of which is depicted in Fig. 6A.

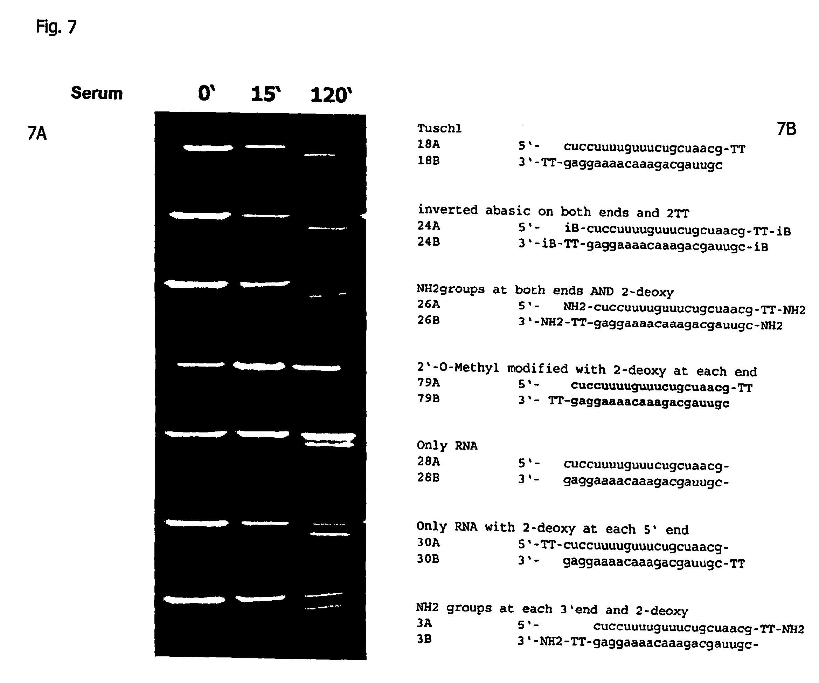

Fig. 7 shows the result of studies on the stability in serum conferred to RNAi molecules by 2'-O-methylation and that end modifications have no beneficial effects on RNAi stability. More particularly, Fig. 7A shows the result of a gel electrophoresis of the various RNAi molecules depicted in Fig. 7B being subject to incubation with fetal calf serum.

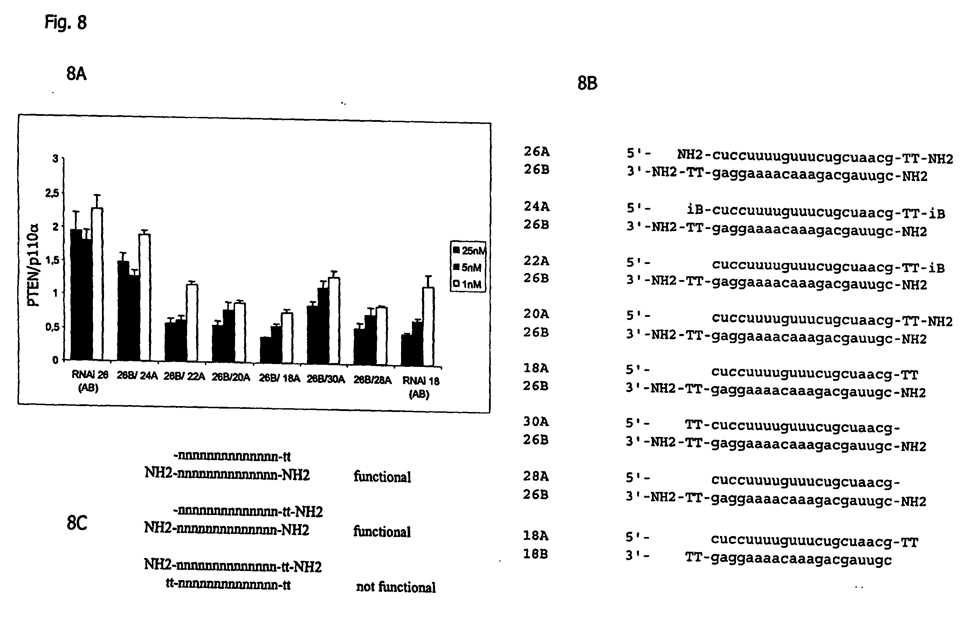

Fig. 8 shows that an amino end modification results in loss of activity. Fig. 8B shows the particular RNAi molecules used in the experiments the result of which is shown in Fig. 8A. Fig. 8C shows the design principles which may be deduced from the results depicted in Fig. 8A. As used in Fig. 8C the term functional means functionally active in the particular assay system as described in the example and "not functional" means not functionally active in said system.

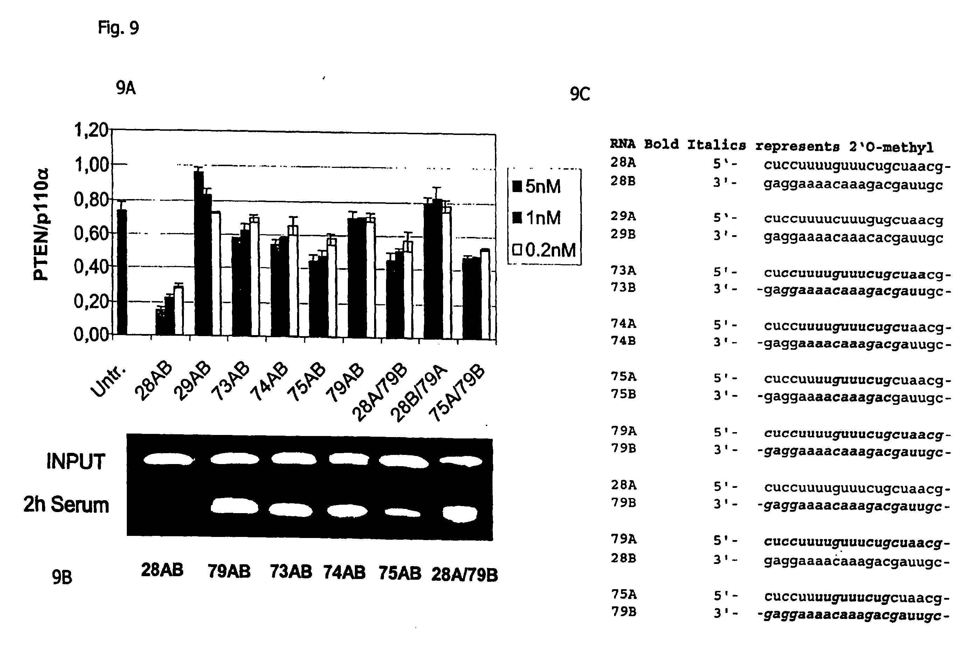

Fig. 9 shows that 2'-O-Alkyl (methyl) modifications stabilize RNAi molecules but also result in reduction of their activity. More particularly, Fig. 9C shows the sequence of the RNAi molecules used in the experiment the result of which is depicted as a dose response curve in Fig. 9A. Fig. 9B shows the result of a gel electrophoresis of the various RNAi molecules depicted in Fig. 9C being subject to a two hour incubation in fetal calf serum.

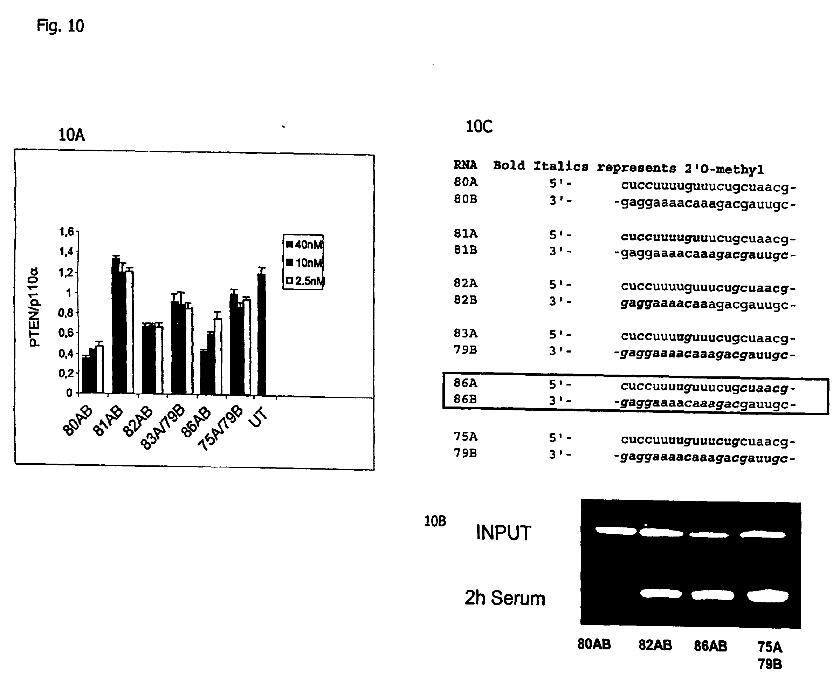

Fig. 10 shows the result of an experiment on the efficacy of RNAi molecules with blocks of 2'-O-methyl modifications with Fig. 10A graphically depicting the results of said experiments as a dose response curve and with Fig. 10C showing the sequences of the particular RNAi molecules used in said experiments. Fig. 10B shows the result of a gel electrophoresis of the various RNAi molecules depicted in Fig. 10C being subject to a two hour incubation in fetal calf serum.

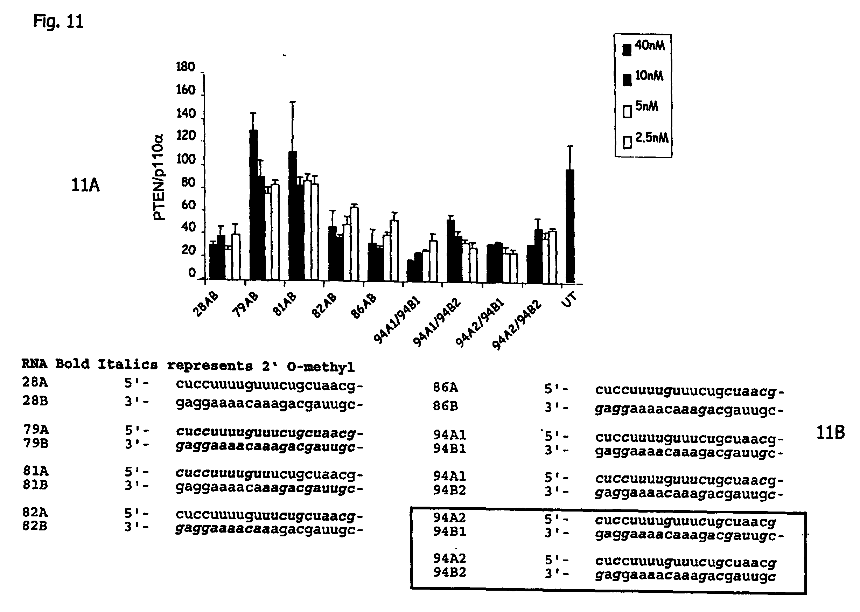

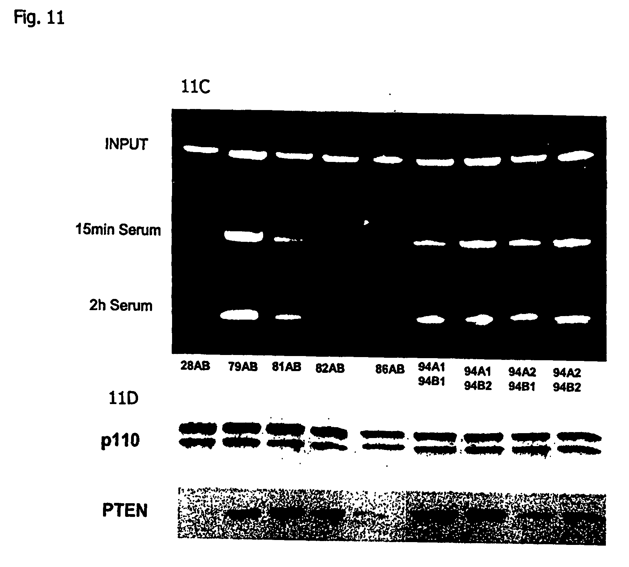

Fig. 11 shows that alternating 2'-O-methyl modification result in activity of the modified RNAi molecules compared to unmodified forms. More particularly, Fig. 11B shows the sequence of the RNAi molecules used in this experiment the result of which is depicted in Fig. 11A. Fig. 11C shows the stability of said RNAi molecules following incubation in serum for two hours, whereas Fig. 11D shows an immunoblot for PTEN protein upon application of different RNAi molecules to HeLa cells. As may be taken therefrom RNAi molecules with alternating modifications are stabilized against endonuclease degradation and active in mediating a PTEN protein knock down.

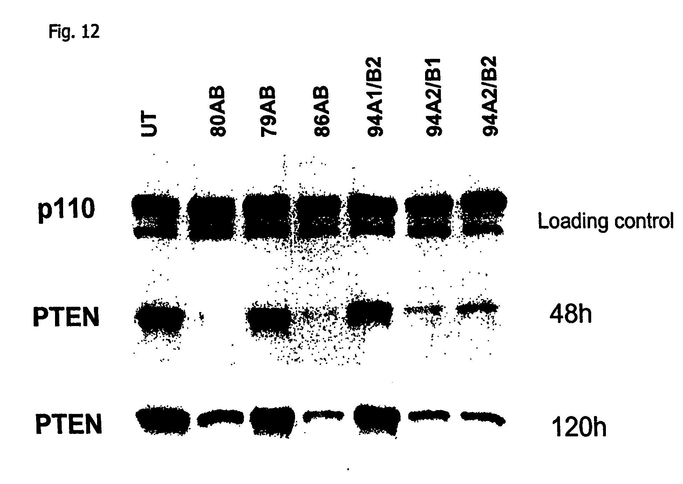

Fig. 12 shows the result of a Western Blot analysis to determine the time course of PTEN protein knock down. Cells were continuously transfected with 2'-O-Methyl modified versus unmodified RNAi molecules using cationic lipids for 72 h. Protein lysates were prepared and analysed by immunoblot after 48 and 120 h. For the 96 h and 120 h timepoints the cells were splitted, replated and incubated in the absence of RNAi molecules for additional 24 and 48 h..

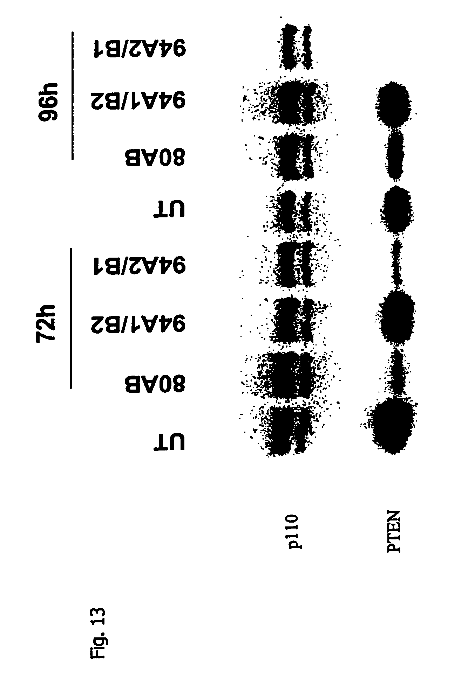

Fig. 13 shows a Western Blot depicting the protein knock down of PTEN being persistent using alternating modified RNAi molecules versus unmodified RNAi molecules. Transfection were performed for only 5 h and new medium without transfection reagents was added. Lysates were analysed by immunoblot 72h and 96h post transfection with the indicated RNAi molecules..

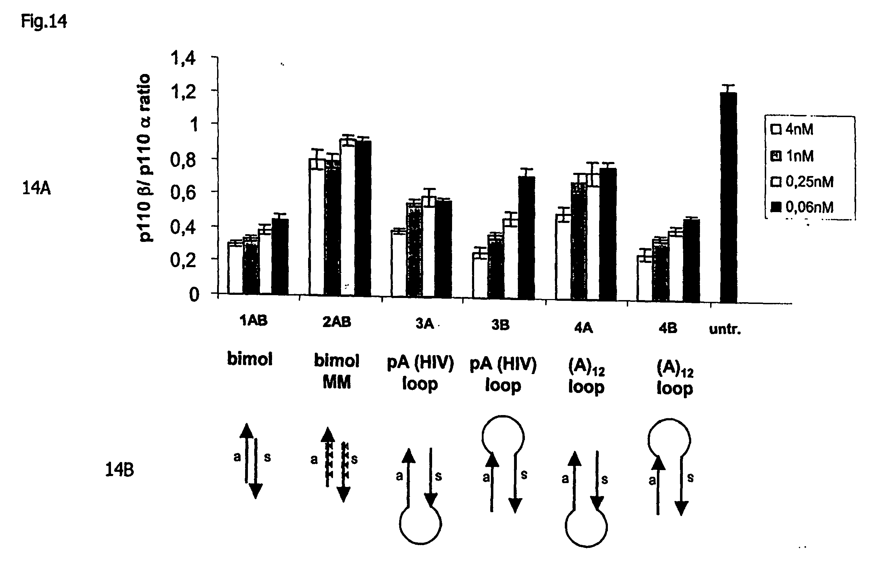

Fig. 14 shows the efficacy of various RNAi molecules with hairpin structures as dose response curve while Fig. 14B shows the structure of said RNAi molecules the result of which is depicted in Fig. 14A. Synthetic siRNAs with different loops are functional in reducing the p110β, Akt1 and Akt 2 expression. (14A) Inhibition of p110β mRNA expression in siRNA transfected HeLa cells. Samples were analyzed in parallel for the level of p110β mRNA expression 24h after transfection of the indicated siRNAs. The transfected bimolecular siRNAs (21mer with 3' TT overhangs, molecule 1AB) or the monomolecular siRNAs with loop structures are schematically shown. Note that the position of the loops (HIV derived pA-loop; (A)₁₂-loop) relative to the antisense sequence is reversed in 3A, 4A relative to 3B, 4B. The 2AB siRNA molecule contains 6 mismatches in the 21mer duplex and serves as a negative control together with the untreated sample. RNA was prepared und subjected to real time RT-PCR (Taqman) analysis. p110β mRNA levels are shown relative to the mRNA levels of p110α, which serve as an internal reference. Each bar represents triplicate transfections (± standard deviation). HeLa cells were transfected at 50% confluency (2500 cells per 96 well) with siRNAs at the indicated concentrations in growth medium.

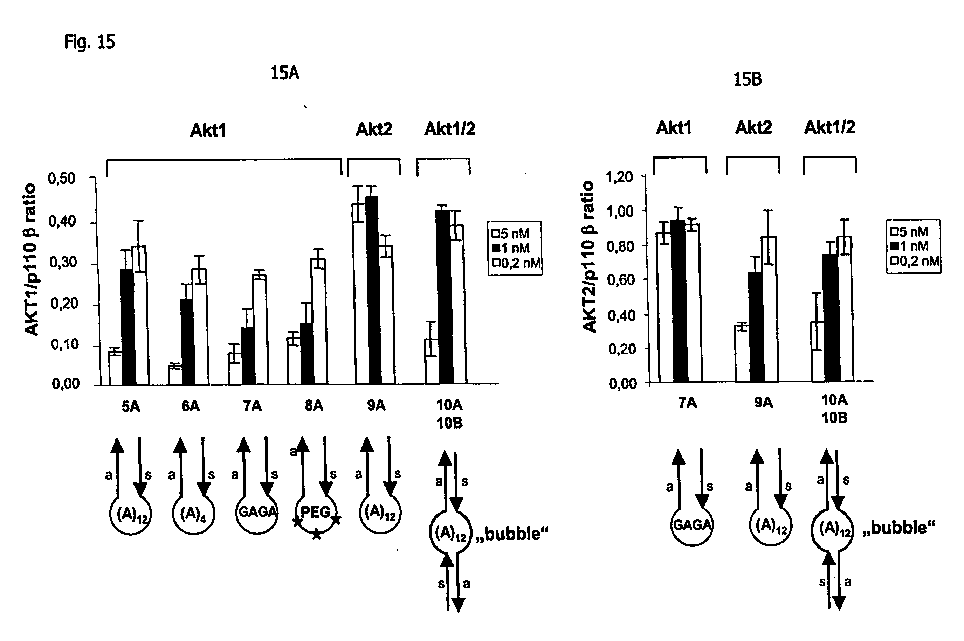

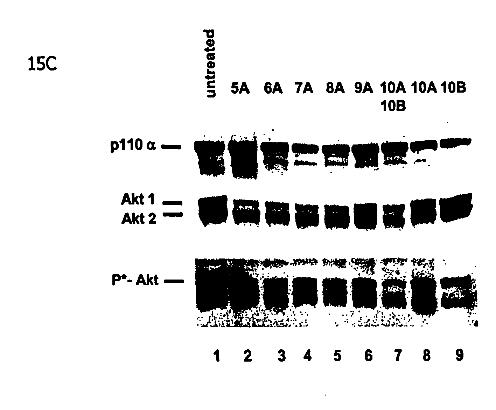

Fig. 15 shows the efficacy of various RNAi molecules with intermolecular and intramolecular loop structures as dose response curves. (15A) Inhibition of Akt1 mRNA expression in siRNA transfected HeLa cells. Samples were analysed in parallel for the level of Akt1 and Akt2 mRNA expression 24h after transfection of the indicated siRNAs. The different loops (A-loops; GAGA-loop and a polyethylenglycol (PEG)-linker) and their putative secondary structure are shown schematically. The siRNA molecule 9A is specific for Akt2 and serves as a negative control. Note that 10A and 10B do not contain self-complementary sequences and are transfected in combination. Akt1 mRNA levels is shown relative to the mRNA levels of p110β, which served as internal control. (**15B**) Inhibition of Akt2 mRNA expression in HeLa cells transfected with the indicated siRNA molecules. Akt2 mRNA levels is shown relative to the mRNA levels of p110β. The Akt1 specific molecule 7A serves here as a negative control.

Fig. 15 C shows a Western Blot analysis on Akt protein depicting the functionality of synthetic siRNAs with different loops in specifically reducing the Akt1 and Akt 2 expression. Inhibition of Akt1 and Akt2 protein expression were analysed by immunoblot. The cells were harvested 48h after transfection of the indicated hairpin siRNAs (20nM). Cell extracts were separated by SDS-PAGE and analysed by immunoblotting using anti-p110 antibody, anti Akt 1/2. Similar results were obtained with an antibody specific for the phosphorylated form of Akt1. The positions of p110α, another catalytic subunit of PI 3-kinase, which was used as a loading control, and of Akt1, Akt2 and phosphorylated Akt (P*-Akt) are indicated on the left.

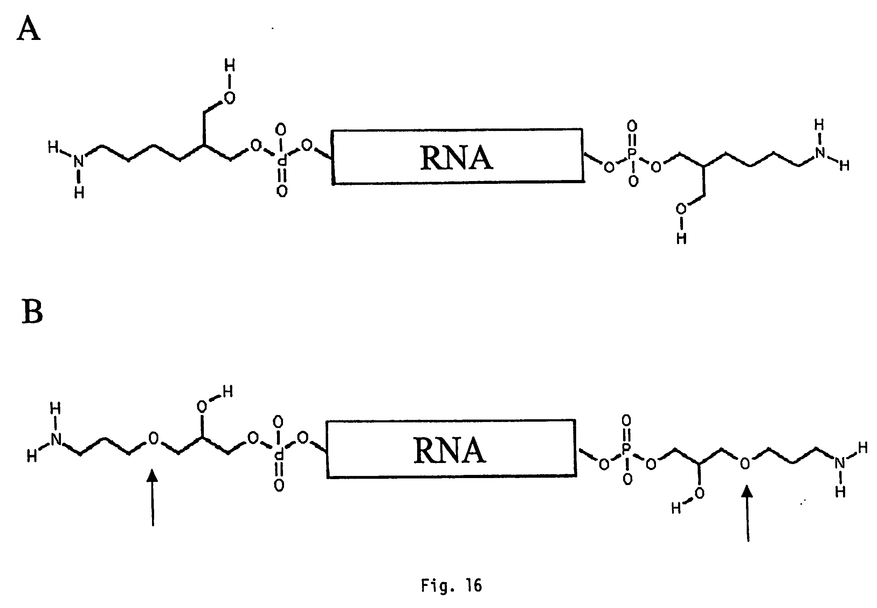

Fig. 16 shows an NH₂ modification, also referred to herein as amino modification, which may be present at either the 3'-OH terminal nucleotide or the 5' terminal nucleotide. The amino group is attached to the phosphate which in turn is attached to the OH group of the sugar moiety, through an alkyl group comprising an alkyl chain of 1 to 8, preferably 6 C atoms, whereby the second C atom close to the phosphate group has a CH₂OH group attached thereo. As an alternative the linker may be formed by an ether whereby the ether is comprised of two alcohols whereby one alcohol is an amino alcohol and the other is a dialcohol with one alcohol group involved in the formation of the ether group and the other one being an OH group located at either of the C atoms, preferably at the second C atom relative to the phosphate group.

### Example 1: Dose response of synthetic duplex RNAi molecules

In this example the impact of NH₂ end protection groups on the activity of duplex RNAi molecules was investigated. Synthetic siRNAs were purchased from Biospring (Frankfurt, Germany). The ribo-oligonucleotides were resuspended in RNase free TE to a final concentration of 50 µM. In the case of bimolecular siRNA molecules equal aliquots (100 µM) were combined to a final concentration of 50 µM. For the formation of intramolecular duplexes the siRNAs were incubated at 50 °C for 2 min in annealing buffer (25mM NaCl; 5mM MgCl₂) and were cooled down to RT. Transfections were carried out in 96 well or 10-cm plates (at 30% to 50% confluency) by using various cationic lipids such as Oligofectamine, Lipofectamine (Life Technologies), NC388 (Ribozyme Pharmaceuticals, Inc., Boulder, CO), or FuGene 6 (Roche) according to the manufacturer's instructions. RNAi molecules were transfected by adding pre-formed 5x concentrated complex of annealed RNAi and lipid in serum-free medium to cells in complete medium. Prior to transfection 2500 HeLa cells were plated per well 15 - 18 hours before transfection for the 96 well format.

The total transfection volume was 100 µl for cells plated in 96-wells and 10 ml for cells in 10 cm plates. The final lipid concentration was 0.8 to 1.2 µg/ml depending on cell density; the RNAi concentration is indicated in each experiment.

Complex formation was allowed to take place for 30min at 37°C. Complexes were added to cells to yield a final 1x concentration of both lipid and RNAi. Depending on the analysis performed following transfection, cells were lysed using a standard cell lysis buffer for protein extraction (Klippel, A., Escobedo, J. A., Hirano, M. and Williams, L. T. (1994). Mol Cell Biol 14, 2675-2685) or a denaturing buffer for RNA isolation according to the RNA isolation kit (Invitek, Berlin (Germany) 24 to 48 hours post transfection for RNA analysis and 48 to 72 hours post transfection for protein analysis by Western Blot.

### Determination of the relative amounts of RNA levels by Taqman analysis:

24h post transfection the RNA of cells transfected in 96-wells was isolated and purified using the Invisorb RNA HTS 96 kit (In Vitek GmbH, Berlin). Inhibition of PTEN mRNA expression was detected by real time RT-PCR (Taqman) analysis using 300 nM PTEN 5' primer CACCGCCAAATTTAACTGCAGA, 300 nM PTEN 3' primer AAGGGTTTGATAAGTTCTAGCTGT and 100 nM of the PTEN Taqman probe Fam-TGCACAGTATCCTTTTGAAGACCATAACCCA-Tamra in combination with 40 nM β-actin 5' primer GTTTGAGACCTTCAACACCCCA, 40 nM β-actin 3' primer GACCAGAGGCATACAGGGACA and 100 nM of the β-actin Taqman probe Vic-CCATGTACGTAGCCATCCAGGCTGTG-Tamra. The Akt primers and probes are determined in Stemberger et al. (Stemberger, a.a.O.) and are used according to the manufacturer's instructions (Applied Biosystem; use of Amplicon Set). Also said primers and probes may be designed usin the software program Primer Express (Applied Biosystem). The reaction was carried out in 50 µl and assayed on the ABI PRISM 7700 Sequence detector (Applied Biosystems) according to the manufacturer's instructions under the following conditions: 48°C for 30 min, 95°C for 10 min, followed by 40 cycles of 15 sec at 95°C and 1 min at 60°C.

RNA knockdown is shown by real time RT-PCR analysis of Hela cells transfected with 21 nt long siRNA duplex molecules unmodified and modified with either NH₂ or inverted Abasics groups at the 5'-end at a lipid carrier concentration of 1.0 µg/ml. Cell density was 2000 cells/well. Modifications on the 3'-end are either RNA overhangs, RNA overhangs with amino groups or DNA overhangs.

**Preparation of cell extracts and immunoblotting.** Cells were washed twice with cold phosphate-buffered saline and lysed at 4°C in lysis buffer containing 20 mM Tris (pH 7.5), 137 mM NaCl, 15% (vol/vol) glycerol, 1% (vol/vol) Nonidet P-40 (NP-40), 2 mM phenylmethylsulfonyl fluoride, 10 mg aprotinin per ml, 20 mM leupeptin, 2 mM benzamidine, 1 mM sodium vanadate, 25 mM β-glycerol phosphate, 50 mM NaF and 10 mM NaPPi. Lysates were cleared by centrifugation at 14,000 x g for 5 minutes and aliquots of the cell extracts containing equal amounts of protein were analyzed for protein expression by Western-blotting: Samples were separated by SDS-PAGE and transferred to nitrocellulose-filters (Schleicher & Schuell). Filters were blocked in TBST buffer (10 mM Tris-HCl (pH 7.5), 150 mM NaCl, 0.05% (vol/vol) Tween 20, 0.5% (wt/vol) sodium azide) containing 5% (wt/vol) dried milk. The respective antibodies were added in TBST at appropriate dilutions. Bound antibody was detected using anti-mouse- or anti-rabbit-conjugated horse radish peroxidase (Transduction Laboratories) in TBST, washed, and developed using the SuperSignal West Dura (Pierce) or ECL (Amersham) chemoluminescence substrates (c.f. Stembergeret al. (2002). *Antisense. Nucl. Ac. Drug Dev. in press.*.

**Antibodies.** The murine monoclonal anti-p110 antibody U3A and the murine monoclonal anti-p85 antibody N7B have been described (Klippel et al., 1994, aaO). Rabbit polyclonal anti-Akt and anti-phospho Akt (S473) antibodies were obtained from Cell Signaling Technology. The murine monoclonal anti-PTEN antibody was from Santa Cruz Biotechnology. The PTEN 53 specific antisense molecule, i. e. geneBloc, is described in Stemberg et al. [Stemberger, supra] having the following sequence (ucuccuuTTGTTTCTGcuaacga), whereby the nucleotide depicted in lower case are ribonucleotides whereas the nucleotide in capital letters are desoxyribonucleotides. This antisense molecule is also identical to RNAi 1A without TT.

The results are shown in Fig. 3A and the respective RNAi molecules in Fig. 3B which are directed to the mRNA of PTEN. The nucleotides written in lower case letters represent ribonucleotides whereas capital letters represent desoxyribonucleotides. The term NH₂ indicates that the 3' '-position of the ribonucleotide was modified by an amino group. The RNAi molecules used in this and other examples disclosed herein are also referred to as small interfering RNA molecules siRNA. It is to be noted that in any of the figures contained herein the upper strand is the antisense or first strand, whereas the lower strand is the sense or second strand of the interfering RNA molecule.

As can be taken from Fig. 3A amino end modifications such as amino modification and inverted abasics modification of the terminal OH group of the nucleic acid are as potent as unmodified ends when the modification is located at the 3' end of the antisense strand (see also Fig. 8A; 8B). Therefore chemical modification to stabilize or with other beneficial properties (delivery) will be tolerated without activity loss when located at the 3' OH; especially when the 3'OH is located on an overhanging nucleotide.

For the experiment shown in Fig. 3C similar conditions as outlined above were used. The first strand and the second strand of the RNAi were either modified by a NH₂ group at the 3'-position of the ribose moiety or by an inverted abasic at said positions. The first construct is designated as siRNA-NH₂ (3A3B) the second as siRNA-iB (4A4B). The sequence of both molecules is depicted in Fig. 3B. The term 3A3B indicates that the interfering ribonucleic acid consists of strand 3A as the antisense strand and strand 3B as the sense strand. For reason of comparison an antisense oligonucleotide designated GB53 (Steinberger et al., supra) was generated which was directed against the PTEN mRNA as well. The particularities of this latter experiment were as follows.

As may be taken from Fig. 3C end protected RNAi molecules depicted in Fig.3B are functional in yielding a PTEN protein knockdown.

From this example it can be taken that both end protection groups render RNAi molecules active in knocking down PTEN protein. This inhibition is as efficient as inhibition with antisense constructs but at lower concentrations used which is a clear advantage over the already very powerful antisense technology.

### Example 2: Overhang requirements for RNAi duplex activity in vivo

The experimental procedures were the same as depicted in connection with example 1 except that the PTEN mRNA targeting interfering RNAi molecules were differently designed. The results are shown in Fig. 4A as dose response curves with Fig. 4B showing the particular sequence and modifications of the interfering RNAi molecules used to generate the data depicted in Fig. 4A. The nomenclature is such that, e. g., RNAi 18 is composed of strand 18A as antisense strand and strand 18B as sense strand.

Blunt ended molecules are compared to molecules with 3'-overhangs (RNAi 18) and 5'-overhangs (RNAi 30 and RNAi 31) in their activity to knockdown PTEN mRNA in Hela cells. The activity of blunt ended molecules (RNAi 28) and molecules with 5'-overhangs is comparable to the activity of molecules with 3'-overhangs. This shows that 3'-overhangs are not essential for RNAi activity.

### Example 3: Duplex length requirements of interfering RNA molecules for RNAi activity in vivo

The experimental approach was similar to the one outlined in connection with example 1 except that the interfering RNA molecules were directed against the mRNA of Akt1. The negative control to show the specificity of the RNAi molecules was again p110 mRNA. The experimental results are shown in Fig. 5A with the particularities of the interfering RNAi molecules used being represented in Fig. 5B.

Taqman analysis on Akt RNA from Hela cells transfected with different RNAi molecules shows that the doublestrand duplex of the siRNA molecules has to be longer than 17 base pairs to show activity whereas molecules with 17 base pair long duplexes or shorter are not functional even if sequence-specific overhangs are added. The shortest RNAi molecules successfully tested were 18 to 19 nucleotides or base pairs in length. It is to be noted that the design of the interfering RNA molecule 51A/51B referred to as RNAi 51 corresponds to the one as described in international patent application WO 01/75164. The RNAi molecule 55A/55B comprises a stretch of 17 nucleotides and has a clearly decreased activity in terms of degradation of Akt mRNA.

Given the experimental results, the minimum requirement for optimum RNAi mediated interference is thus a duplex length of 18 or 19 nucleotides, independent of the further design of the RNAi molecules such as blunt end or 5'-overhang or any other form as disclosed herein, but generally applicable to RNAi molecules. However, it has to be acknowledged that the particular design of the RNAi molecules may confer further advantages to said molecules, such as, e. g., increased efficiency and increased stability, respectively.

### Example 4: Target -antisense homology requirements for RNAi in vivo

The experimental set-up was similar to the one described in example 1, whereby the RNAi is specific for Akt1. In addition, a PTEN specific interfering RNA molecule was designed and used as negative control. The results are shown in Fig. 6A and Fig. 6B.

Having established the minimal duplex length of 18 or more than 18 nucleotides for functional siRNA molecules we have asked the question how many matching nucleotides between target mRNA and siRNA are necessary for silencing activity.As shown by Taqman analysis on Akt1 RNA a stretch of 19 to 15 nucleotides perfectly matching to the target RNA, in the present case Akt 1, is sufficient to mediate RNAi activity. A PTEN specific RNAi molecule does not reduce RNA amounts of Akt1 thus confirming the specificity of this approach. Mismatches of one or two nucleotides at any or both ends of a strand are functional suggesting that a homolog stretch of 15 nt between a target mRNA and RNAi is sufficient for gene silencing. It can be concluded from these data that unspecific gene silencing can occur by chance through unspecific binding to unrelated targets. This is based on the understanding that a stretch of 15 to 17 matching base pairs is not specific for a single gene and will occur by chance considering the complexitiy and size of the genome or transcriptosome of vertrebrates.

Based on this observation the present invention is reducing this off-target problem of siRNA by two approaches. First by reducing the molecule length of the siRNA molecules to the minimal requirements (18-19 nt) and thereby reducing the chance of homology to off-targets. Second, by inactivation of the sense strand to prevent a unwanted RNA silencing caused by accidential complemetarity of the sense strand to a unrelated target RNA (see also Example 6).

### Example 5: Stability of modified RNAi molecules in serum

Oligonucleotides were incubated in human serum for 15 min and two hours and loaded on 10% polyacrylamide gel with untreated controls. The results are shown in Fig. 7A. The various RNAi molecules used are shown and described in more detail in Fig. 7B.

From this example it can be taken that the RNAi duplex of RNA molecules with all nucleotides modified with 2'-O-methyl groups (RNAi molecules 79A79B and 28A28B) have higher stability in serum. It is also shown that a blunt duplex is more stable than the duplex molecule with overhangs. From this the conclusion may be drawn that end protection (e.g. iB or Amino) is not increasing the stability in serum.

In addition, it can also be concluded that in contrast to the understanding in the art before the filing of this application endonucleases rather than exonucleases are more important in the protection of RNAi molecules.

In view of this, in addition to the various modifications or designs of the inventive RNAi molecules as disclosed in this application a further or additional modification of the nucleotides may be the use of a phosphorothioate backbone of the RNAi molecules which may be either complete or partial in order to inhibit endonuclease function. A complete phosphorothioate backbone means that any of the nucleotides exhibits a phosphorothioate group whereas a partial phosphorothioate backbone means that not all of the nucleotides forming the RNAi molecule have a phosphorothioate modification. This modification is suitable to increase the lifetime of RNAi molecules irrespective of the further design of RNAi molecules. In this regard, a partially or completely phosphorothioate modified RNAi is subject to the present invention which may be realized in connection with the different strategies for the design of interfering RNA molecules as disclosed herein or with any of the designs known in the art.

### Example 6: Inactivation of the sense strand by NH₂ end protection groups on the 5' and 3' ends

The experimental set-up was similar to the one described in connection with example 1 with the target nucleic acid sequence being PTEN mRNA. The concentration of Hela cells was 2,000 cells per well. RNA of PTEN was analysed in Taqman assays after transfection of differently modified RNAi molecules. The different interfering RNA molecules used are depicted in Fig. 8B whereas the experimental results are shown in Fig. 8A.

As may be taken from the dose response curves of various RNAi molecules depicted in Fig. 8A RNAi molecules are functional when the sense strand, i. e. the second stand, is modified on both ends with amino groups. Particularly effective are RNAi molecules 20A26B, 18A26B, and 28A26B. The lowest activity is shown by RNAi molecule 26A26B which corresponds to end modification on all 4 ends of the duplex (Tuschl is 18AB) .

However, RNAi activity is also achieved when the antisense strand, i. e. the first strand, is modified only at the 3' end leaving a free OH group at the 5' end (RNAi constructs 22A26B; 20A26B). There is no activity when the antisense strand is modified with amino groups on both the 5' and the 3' end (26A26B). This leads to the conclusion that any end of the antisense strand and more particularly the 5' end of the antisense should be kept without modifications. Additionally, it is worth stating that the NH₂ end modification can be used to inactivate the sense strand on the 5' and 3' end and therefore reduce off-target effects mediated by an otherwise functional sense strand which results in a significantly increased specificity of the RNAi molecule which is advantageous for target validation as well as for any medical use of the RNAi molecule.

The further generalisation of the results from this experiment is depicted in Fig. 8C. Functionally active RNAi are accordingly those not having an amino modification at the antisense strand or having an amino modification only at the 3' end of the antisense strand whereas an amino modification at both ends of the antisense strand is not functional, i. e. does not result in a knockdown of the target mRNA.

### Example 7: Impact of 2'-O-methyl modification of RNAi molecules for endonuclease protection.

RNA knockdown is again shown using the real time RT-PCR analysis on Hela cells transfected with RNAi duplex molecules directed against the PTEN mRNA as represented in Fig. 9A. Experimental procedures were basically the same as specified in example 1. The structure of the RNAi molecules investigated and their dose responses, which are depicted in Fig. 9A, are shown in Fig. 9C. The nucleotides printed in bold are those having a 2'-O-methyl modification.

It is illustrated by the dose response curves shown for various RNAi molecules in Fig. 9A that internal 2'-O-alkyl groups are reducing RNAi activity. Preferably such 2'-O-alkyl groups are 2'-O-methyl or 2'-O-ethyl groups. However, molecules with unmodified nucleotides in combination with 2'-O-alkyl modification show significant activity. As is also depicted in Fig. 9A there is no activity when the antisense strand is all modified with 2'-O-methyl groups and the sense strand is not modified (c. f., e. g., RNAi molecule 79A28B). Taken the results of a stability test such as incubation of the various RNAi molecules in serum, as depicted in Fig. 9B, shows that 2'-O-alkyl modifications stabilize RNAi molecules against degradation. This clearly beneficial effect, however, is at least to a certain degree counterbalanced by the effect that 2'-O-alkyl modifications generally result in a reduced knockdown activity. Accordingly, the design of RNAi molecules has to balance stability against activity which makes it important to be aware of the various design principles as disclosed in the present application.

### Example 8: Impact of blocks of internal 2'-O-methyl modifications on the stability of RNAi molecules in serum.

The experimental approach in connection with this study was actually the same as depicted in example 1. Again, PTEN RNA is analysed by real time RT-PCR on Hela cells at a density of 2000 cells/well which were transfected with different doses of RNAi molecules. RNAi molecules were incubated in serum for two hours and analysed on a 10% polyacrylamide gel The results of this study are illustrated in Figs. 10A to 10C, whereby Fig. 10A shows the dose response of the various RNAi molecules depicted in Fig. 10C and Fig. 10B shows the result of a stability test using some of the RNAi molecules depicted in Fig. 10C. It is to be acknowledged that the nucleotides written in bold in Fig. 10C are the ones carrying a modification which is in the present case of a 2'-0-methyl modification of the ribose moiety of the nucleotides.

There is a dose dependent inhibition by the unmodified RNAi molecules. It is also shown that the 2'-O-methyl modification of the core 9 nucleotides makes the RNAi stable in serum and allows activity of the duplex in the meaning of mediating the interference phenomenon leading to a degradation of the PTEN mRNA. Total modification of the sense strand makes the RNAi molecule stable in serum and allows certain activity.

Alternating blocks of 5 nucleotides with 2'-O-methyl modification renders the RNAi molecule stable in serum and allows activity on PTEN RNA as shown by incubating the RNAi duplex in serum for two hours and loading the samples on a 10% polyacrylamide gel. As may be taken from Fig. 10B the duplex comprising strands 80A and 80B is strongly degraded after incubation in serum for two hours. The duplex consisting of strands 82A and 82B confirms the result that the 5'-end of the first strand which comprises the antisense strand should not be modified at the 5'-terminal nucleoties (compare 82A82B with the reverse orientated 81A81B). This is also confirmed by the results obtained having the duplex consisting of the strands 86A and 86B which is both active and stabilised in serum. It is noteworthy that molecules with unmodified blocks at the terminal 5' of the antisense strand are more active whereby the 5 'terminal OH group is preferably not derivatized.

Further experiments were carried out using different modification patterns of 2'O-methyl modification of the nucleotides. The results thereof are shown in Fig. 11 A to 11 C and further discussed herein in example 9.

### Example 9: The impact of alternating internal 2'-O-alkyl modification on serum stability of RNAi molecules.

The experimental set-up for performing this kind of study was the same as used in connection with the studies reported in example 1 and example 8, respectively, with the targeted nucleic acid being again PTEN mRNA. Hela cells were transfected with the different RNAi molecules depicted in Fig. 11B and RNA knockdown was demonstrated using real time RT-PCR on PTEN RNA in a dose-dependent manner (Fig. 11 A). The stability of the various RNAi molecules after 15 min and two hours in serum at 37°C is depicted in Fig. 11C and a Western Blot for p110 and PTEN as the target-protein of the various RNAi molecules is depicted in Fig. 11D with the RNAi molecules tested being the same in both the experiments underlying Fig. 11 C and Fig. 11 D.

As illustrated in Fig. 11A and Fig. 11C nucleotides modified with 2'-O-methyl groups alternating with unmodified nucleotides render RNAi molecules stable in serum while still allowing them to be active in the sense of interfering with the target mRNA. It is shown that incubation of RNAi duplex molecules for 15 min and two hours in serum will degrade the unmodified duplex and the duplex where the 10 most 5'-positioned nucleotides are unmodified.

In the RNAi molecules represented in Fig. 11B various patterns of modified and unmodified nucleotides are realised. The RNAi molecule 94A1/94B1 comprises a structure wherein a modified nucleotide is flanked by an unmodified nucleotide with the unmodified nucleotide being located at the 5' end of the first strand. The RNAi molecule comprised of strands 94A2 and 94B2 is another example where the modified nucleotides and the unmodified nucleotides of the first and the second strand are located at opposing sites. In contrast to this the RNAi molecule comprised of strands 94A1 and 94B2 have the same pattern of modified and unmodified nucleotides. However, there is a phase shift such that the modified nucleotide forms base paring with an unmodified nucleotide. The two RNAi molecules comprised of strands 94A1 and 94B1 and strands 94A2 and 94B2 differ from each other such that in the first case the first strand starts with an unmodified nucleotide and the corresponding first nucleotide of the second strand, i. e. the nucleotide at the 3' end of the second strand, starts with an unmodified nucleotide with the arrangement being opposite to this in the RNAi molecule comprised of 94A2 and 94B2.

Additionally, alternatingly modified RNAi molecules as depicted in Fig. 11B are functional in mediating a PTEN protein knock down as shown in Fig. 11D but only when the second 5' and second 3' terminal nucleotide is not modified (see 94A294B1 and 94A294B2). Taken together these data show that the most stable and most active RNAi molecules do have alternating 2'Alkyl modified and unmodified nucleotide residues. It should be noted that these molecules do show a very similar mRNA reduction when compared to unmodified siRNA molecules which being stable in serum allows increased or easier handling.

### Example 10: Functional Protein Knockdown mediated by internally modified RNAi molecules

The experimental approach was similar to the one outlined in connection with example 1.

Western Blots were performed on HeLa cells harvested at various time points following transfection (48, 72, 96 and 120 hours) with alternatingly modified RNAi molecules as depicted in Fig. 11 B. For experimental reasons it is noteworthy that at the 96 hour time point cells have been split and half the population was replated. A total of 40 nM of the various RNAi molecules were applied to the cells. Cells were continuously transfected for 72h with cationic lipids as described in example 1; then replated in the absence of transfection reagents.

Transfections were carried out in 96 well or 10-cm plates (at 30% to 50% confluency) by using various cationic lipids such as Oligofectamine, Lipofectamine (Life Technologies), NC388, L8 (Atugen, Berlin), RNAi were transfected by adding pre-formed 5x concentrated complex of siRNAs and lipid in serum-free medium to cells in complete medium. The total transfection volume was 100 µl for cells plated in 96-wells and 10 ml for cells in 10 cm plates. The final lipid concentration was 0.8 to 1.2 µg/ml depending on cell density; the siRNA concentration is indicated in each experiment.

The result of the Western Blot analysis is depicted in Fig. 12. As can be taken from this Figure, modified RNAi molecules of the 94A2B 1 and 94A2B2 version yield a longer lasting PTEN protein knock down as unmodified molecules. The lack of protein knock down also seen in Fig. 11 with molecules of the 94A1B1 and 94A1B2 version is confirmed in this experiment. Unmodified molecules (80AB) are not as potent in supporting long lasting protein knock down when the cells are not continuously transfected.

### Example 11: Persistent PTEN protein knock down with alternating 2'-O-methyl modifications of RNAi molecules

The experimental approach was similar to the one outlined in connection with example 10 with the exception that the transfection was terminated after 5 h by replacing the transfection medium with new medium.The protocol was slightly modified such that for each of the RNAi molecules a 40 nM concentration was realized using a stock solution of 1 µg RNAi / ml cationic lipid as described in connection with example 1. 5 hours after transfection the medium was withdrawn and fresh EMEM added. The cells were split after 72, with half of the cells being lysed and the other half newly plated and lysed 24 h later (96h post transfection). The result of a Western Blot analysis using 3 different RNAi molecules (80AB, 94A1/B2, 94A2/B 1 are depicted in Fig. 13. As a positive control untreated cells were used. Fig. 13 shows the expression of PTEN after 72 h and 96 h, respectively. Taken the structural particularities of the various RNAi molecules it can be taken from Fig. 13 that protein knockdown is persistent with alternating molecules of the 94A2B 1 kind even over 96 h after splitting and replating cells compared to unmodified RNAi molecules (such as 80AB) and RNAi molecule 94A1B2.

### Example 12: Different loop structures are functional in mediating RNA interference

To test whether RNAi molecules, preferably synthetic RNAi molecules with self complementary structures can inhibit gene expression as efficiently as standard double stranded siRNA molecules, HeLa cells were transfected with p110β specific synthetic siRNAs. Transfections were carried out in 96 well or 10-cm plates (at 30% to 50% confluency) by using various cationic lipids such as Oligofectamine, Lipofectamine (Life Technologies), GeneBlocs were transfected by adding pre-formed 5x concentrated complex of GB and lipid in serum-free medium to cells in complete medium. The total transfection volume was 100 µl for cells plated in 96-wells and 10 ml for cells in 10 cm plates. The final lipid concentration was 0.8 to 1.2 µg/ml depending on cell density; the RNAi molecule concentration is indicated in each experiment.

A dose dependent titration showed no significant difference in efficiency of mRNA knock-down achieved by the standard bimolecular double strand 21mer and the corresponding monomolecular molecules as analysed by realtime PCR (Taqman) (Fig. 14A). Two different loop structures a (A)₁₂ loop and a HIV derived pA-loop were tested in parallel with similar results. A comparision of the relative position of the antisense sequence and the loop structure revealed an improved knock-down efficiency with the antisense sequence being located 3' to the loop (Fig. 14B; compare construct 3A, 3B and 4A, 4B).

### Example 13: Studies on the efficacy of different intramolecular hairpin loops and intermolecular "bubbles"

The influence of different loop structures on inhibition of mRNA and protein expression was tested. For these experiments Akt1 and Akt2 were chosen as targets. The experimental approach was similar to the one described in example 12.

Significantly, the reduction in Akt1 mRNA as depicted in Fig. 15A and Fig. 15B as well as Akt1 protein levels as depicted in Fig. 15C was completely independent of the loop structure tested (compare molecules 5A, 6A, 7A, 8A) (the structure of the RNAi molecule tested is always depicted underneath the bar diagram). Even a molecule containing a rather unphysiological structure such as a polyethylenglycol linker (PEG) as a loop efficiently reduced Akt1 expression indicating that size and nucleotide sequence of the loop is not crucial (Fig. 15A; molecule 8A). A synthetic siRNA molecule specific for Akt2 (9A) was used to control for specificity, and had no effect on Akt1 levels as shown in Fig. 15A. This molecule, however, efficiently silenced Akt2 expression (Fig. 15B; Fig. 15C). Self-complementary RNA molecules with loop structures have the possibility to anneal as doublestrands in monomolecular or bimolecular structures under physiological hybridization conditions (Fig. 15B, loop or bubble structure). To address the question of whether the siRNA molecules exert their function via adapting an intra-molecular loop or an inter-molecular "bubble" (schematically shown in Fig. 15B) two molecules not capable of folding back on themselves were transfected. These constructs contained Akt1- and Akt2-specific sequences within the same molecule (Fig. 15B, constructs 10A, 10B) and were designed to be restricted to form a bimolecular duplex ("bubble"). Surprisingly, this molecule efficiently mediated both Akt1 and Akt2 mRNA knock-down as well as protein knock-down when transfected after annealing of both strands.

Whether loop and bubble structures are indeed substrates for RNA processing enzymes, e.g. *Dicer,* is not clear at this point. A recent study by Paddison and coworkers suggests that hairpin containing siRNAs are more dependent on *Dicer* activity than double stranded siRNAs. However, these data demonstrating RNA interference activity using a PEG linker molecule indicate that the linker sequence is likely to be irrelevant.

## Claims

1. A ribonucleic acid mediating RNA interference comprising a double stranded structure whereby the double- stranded structure comprises a first strand and a second strand, whereby the first strand comprises a first stretch of contiguous nucleotides and whereby said first stretch is at least partially complementary to a target nucleic acid, and the second strand comprises a second stretch of contiguous nucleotides whereby said second stretch is at least partially identical to the target nucleic acid, **characterised in that** the double stranded structure is blunt ended on both sides of the double strand and the length of said first strand and said first stretch and the length of said second strand and said second stretch is 18 or 19 bases, for use in a method for the treatment of a disease.

2. The ribonucleic acid according to claim 1, wherein at least one nucleotide of the ribonucleic acid has a modification at the 2'-position and the modification is preferably selected from the group comprising amino, fluoro, methoxy and alkyl.

3. The ribonucleic acid according to any of claims 1 to 2, wherein the complementarity between said first strand and the target nucleic acid is perfect.

4. The ribonucleic acid according to any of claims 1 to 2, wherein the duplex formed between the first strand and the target nucleic acid comprises at least 15 nucleotides wherein there is one mismatch or two mismatches between said first strand and the target nucleic acid forming said double-stranded structure.

5. The ribonucleic acid according to any of claims 1 to 4, wherein the target gene is selected from the group comprising structural genes, housekeeping genes, transcription factors, motility factors, cell cycle factors, cell cycle inhibitors, enzymes, growth factors, cytokines and tumor suppressors.

6. The nucleic acid according to any of claims 1 to 5, wherein the disease is selected from the group comprising glioblastoma, prostate cancer, breast cancer, lung cancer, liver cancer, colon cancer, pancreatic cancer and leukaemia, diabetes, obesity, cardiovascular diseases, and metabolic diseases.

7. Use of a ribonucleic acid according to any of claims 1 to 6 for the manufacture of a medicament.

8. The use according to claim 7, wherein the medicament is for the treatment of a disease or of a condition which is selected from the group comprising glioblastoma, prostate cancer, breast cancer, lung cancer, liver cancer, colon cancer, pancreatic cancer and leukaemia, diabetes, obesity, cardiovascular diseases, and metabolic diseases.

9. A cell, preferably a knockdown cell, containing a ribonucleic acid according to any of claims 1 to 6, whereby if the cell is a human cell, the human cell is an isolated human cell.

10. An organism, preferably a knockdown organism, containing a ribonucleic acid according to any of claims 1 to 8, whereby the organism is different from a human being.

11. A composition containing a ribonucleic acid according to any of claims 1 to 6.

12. A pharmaceutical composition containing a ribonucleic acid according to any of claims 1 to 6 and a pharmaceutically acceptable carrier.

## Patentansprüche

1. RNA-Interferenz vermittelnde Ribonukleinsäure, umfassend eine doppelsträngige Struktur, wobei die doppelsträngige Struktur einen ersten Strang und einen zweiten Strang umfasst, wobei der erste Strang einen ersten Abschnitt aufeinanderfolgender Nukleotide umfasst und wobei der erste Abschnitt zu einer Ziel-Nukleinsäure wenigstens teilweise komplementär ist, und der zweite Strang einen zweiten Abschnitt aufeinanderfolgender Nukleotide umfasst, wobei der zweite Abschnitt wenigstens teilweise mit der Ziel-Nukleinsäure identisch ist, **dadurch gekennzeichnet, dass** die doppelsträngige Struktur an beiden Seiten des Doppelstranges ein glattes Ende aufweist und die Länge des ersten Strangs und des ersten Abschnitts und die Länge des zweiten Strangs und des zweiten Abschnitts 18 oder 19 Basen beträgt, zur Verwendung in einem Verfahren zur Behandlung einer Krankheit.

2. Ribonukleinsäure nach Anspruch 1, wobei wenigstens ein Nukleotid der Ribonukleinsäure eine Modifikation an der 2'-Position aufweist und die Modifikation bevorzugterweise ausgewählt ist aus der Gruppe umfassend Amino, Fluor, Methoxy und Alkyl.

3. Ribonukleinsäure nach einem der Ansprüche 1 bis 2, wobei die Komplementarität zwischen dem ersten Strang und der Ziel-Nukleinsäure perfekt ist.

4. Ribonukleinsäure nach einem der Ansprüche 1 bis 2, wobei der zwischen dem ersten Strang und der Ziel-Nukleinsäure gebildete Duplex wenigstens 15 Nukleotide umfasst, wobei es eine Fehlpaarung oder zwei Fehlpaarungen zwischen dem ersten Strang und der Ziel-Nukleinsäure, die die doppelsträngige Struktur bilden, gibt.

5. Ribonukleinsäure nach einem der Ansprüche 1 bis 4, wobei das Zielgen ausgewählt ist aus der Gruppe umfassend Strukturgene, Haushaltsgene, Transkriptionsfaktoren, Motilitätsfaktoren, Zellzyklusfaktoren, Zellzyklusinhibitoren, Enzyme, Wachstumsfaktoren, Zytokine und Tumorsuppressoren.

6. Nukleinsäure nach einem der Ansprüche 1 bis 5, wobei die Krankheit ausgewählt ist aus der Gruppe umfassend Glioblastom, Prostatakrebs, Brustkrebs, Lungenkrebs, Leberkrebs, Kolonkrebs, Bauchspeicheldrüßenkrebs und Leukämie, Diabetes, Obesität, kardiovaskuläre Erkrankungen und Stoffwechselerkrankungen.

7. Verwendung einer Ribonukleinsäure nach einem der Ansprüche 1 bis 6 zur Herstellung eines Medikaments.

8. Verwendung nach Anspruch 7, wobei das Medikament für die Behandlung einer Krankheit oder eines Zustandes ist, die/der ausgewählt ist aus der Gruppe umfassend Glioblastom, Prostatakrebs, Brustkrebs, Lungenkrebs, Leberkrebs, Kolonkrebs, Bauchspeicheldrüßenkrebs und Leukämie, Diabetes, Obesität, kardiovaskuläre Erkrankungen und Stoffwechselerkrankungen.

9. Zelle, bevorzugterweise eine Knock-Down-Zelle, enthaltend eine Ribonukleinsäure nach einem der Ansprüche 1 bis 6, wobei wenn die Zelle eine humane Zelle ist, die humane Zelle eine isolierte humane Zelle ist.

10. Organismus, bevorzugterweise ein Knock-Down-Organismus, enthaltend eine Ribonukleinsäure nach einem der Ansprüche 1 bis 8, wobei der Organismus von einem Menschen verschieden ist.

11. Zusammensetzung, umfassend eine Ribonukleinsäure nach einem der Ansprüche 1 bis 6.

12. Pharmazeutische Zusammensetzung, umfassend eine Ribonukleinsäure nach einem der Ansprüche 1 bis 6 und einen pharmazeutisch annehmbaren Träger.

## Revendications

1. Acide ribonucléique médiant l'ARN interférence, comprenant une structure double brin, la structure double brin comprenant un premier brin et un deuxième brin, le premier brin comprenant un premier segment de nucléotides contigus, et ledit premier segment étant au moins partiellement complémentaire d'un acide nucléique cible, et le deuxième brin comprenant un deuxième segment de nucléotides contigus, ledit deuxième segment étant au moins partiellement identique à l'acide nucléique cible, **caractérisé en ce que** la structure double brin possède une extrémité franche sur les deux côtés du double brin, et la longueur dudit premier brin et dudit premier segment, et la longueur dudit deuxième brin et dudit deuxième segment, étant de 18 ou 19 bases, pour utilisation dans un procédé de traitement d'une maladie.

2. Acide ribonucléique selon la revendication 1, dans lequel au moins un nucléotide de l'acide ribonucléique possède une modification sur la position 2', la modification étant de préférence choisie dans le groupe comprenant les modifications amino, fluoro, méthoxy et alkyle.

3. Acide ribonucléique selon l'une quelconque des revendications 1 et 2, dans lequel la complémentarité entre ledit premier brin et l'acide nucléique cible est parfaite.

4. Acide ribonucléique selon l'une quelconque des revendications 1 et 2, dans lequel le duplex formé entre le premier brin et l'acide nucléique cible comprend au moins 15 nucléotides, un mésappariement ou deux mésappariements existant entre ledit premier brin et l'acide nucléique cible formant ladite structure double brin.

5. Acide ribonucléique selon l'une quelconque des revendications 1 à 4, dans lequel le gène cible est choisi dans le groupe comprenant les gènes de structure, les gènes domestiques, les facteurs de transcription, les facteurs de motilité, les facteurs de cycles cellulaires, les inhibiteurs de cycles cellulaires, les enzymes, les facteurs de croissance, les cytokines et les suppresseurs tumoraux.

6. Acide nucléique selon l'une quelconque des revendications 1 à 5, dans lequel la maladie est choisie dans le groupe comprenant le glioblastome, le cancer de la prostate, le cancer du sein, le cancer du poumon, le cancer du foie, le cancer du côlon, le cancer du pancréas et la leucémie, le diabète, l'obésité, les maladies cardiovasculaires et les maladies métaboliques.

7. Utilisation d'un acide ribonucléique selon l'une quelconque des revendications 1 à 6, pour la fabrication d'un médicament.

8. Utilisation selon la revendication 7, pour laquelle le médicament est destiné au traitement d'une maladie ou d'un état pathologique qui est choisi dans le groupe comprenant le glioblastome, le cancer de la prostate, le cancer du sein, le cancer des poumons, le cancer du foie, le cancer du côlon, le cancer du pancréas et la leucémie, le diabète, l'obésité, les maladies cardiovasculaires et les maladies métaboliques.

9. Cellule, de préférence cellule knockdown, contenant un acide ribonucléique selon l'une quelconque des revendications 1 à 6, la cellule humaine étant une cellule humaine isolée si la cellule est une cellule humaine.

10. Organisme, de préférence organisme knockdown, contenant un acide ribonucléique selon l'une quelconque des revendications 1 à 8, l'organisme étant différent d'un être humain.

11. Composition contenant un acide ribonucléique selon l'une quelconque des revendications 1 à 6.

12. Composition pharmaceutique contenant un acide ribonucléique selon l'une quelconque des revendications 1 à 6 et un support pharmaceutiquement acceptable.
